# EUROPEAN PATENT APPLICATION

(11) **EP 1 693 448 A1**
(43) Date of publication of application: **23.08.2006**
(21) Application number: 03751478.3
(22) Date of filing: 14.10.2003
(51) Int. Cl.: C12N 15/09, C07K 16/18, A61K 39/395, A61P 7/00, A61P 31/12, A61P 35/00, A61P 37/00

(54) **DOUBLE SPECIFIC ANTIBODIES SUBSTITUTING FOR FUNCTIONAL PROTEIN**

(71) Applicant: CHUGAI SEIYAKU KABUSHIKI KAISHA, Tokyo, 115-8543 (JP)
(72) Inventor: HATTORI, Kunihiro, CHUGAI SEIYAKU KABUSHIKI KAISHA, Gotenba-shi, Shizuoka 412-8513 (JP); KOJIMA, Tetsuo, CHUGAI SEIYAKU KABUSHIKI KAISHA, Gotenba-shi, Shizuoka 412-8513 (JP); MIYAZAKI, Taro, CHUGAI SEIYAKU KABUSHIKI KAISHA, Gotenba-shi, Shizuoka 412-8513 (JP); SOEDA, Tetsuhiro, CHUGAI SEIYAKU KABUSHIKI KAISHA, Gotenba-shi, Shizuoka 412-8513 (JP); SENOO, Chiaki, CHUGAI SEIYAKU KABUSHIKI KAISHA, Gotenba-shi, Shizuoka 412-8513 (JP); NATORI, Osamu, CHUGAI SEIYAKU KABUSHIKI KAISHA, Gotenba-shi, Shizuoka 412-8513 (JP); KASUTANI, Keiko, CHUGAI SEIYAKU KABUSHIKI KAISHA, Gotenba-shi, Shizuoka 412-8513 (JP); ISHII, Shinya, CHUGAI SEIYAKU KABUSHIKI KAISHA, Gotenba-shi, Shizuoka 412-8513 (JP)
(74) Representative: Vossius & Partner
(86) International application number: PCT/JP2003/013123
(87) International publication number: WO 2005/035754

(57) **Abstract**

The present inventors succeeded in separating bispecific antibodies that functionally substitute for ligands of type I interferon receptors comprising two types of molecules: AR1 chain and AR2 chain. Furthermore, the present inventors succeeded in producing bispecific antibodies that substitute for the enzyme reaction-accelerating function of blood coagulation factor VIII/activated blood coagulation factor VIII, which bind to both blood coagulation factor IX/activated blood coagulation factor IX and blood coagulation factor X.

SEQUENCE LISTING

<110>  CHUGAI SEIYAKU KABUSHIKI KAISHA

<120>  BISPECIFIC ANTIBODY SUBSTITUTING FOR FUNCTIONAL PROTEINS

<130>  C1-A0313P2

<140>  PCT/JP2003/013123
<141>  2003-10-14

<160>  82

<170>  PatentIn version 3.1

<210>  1
<211>  120
<212>  PRT
<213>  Homo sapiens

<400>  1
Gln Val Gln Leu Lys Gln Ser Gly Ala Glu Leu Val Arg Pro Gly Ala
1               5                   10                  15

Ser Val Arg Leu Ser Cys Lys Ala Ser Gly Tyr Thr Phe Thr Phe Tyr
            20                  25                  30

Trp Ile Asn Trp Ile Lys Gln Arg Pro Glu Gln Gly Leu Glu Trp Ile
        35                  40                  45

Gly Arg Ile Asp Pro Tyr Asp Ser Glu Thr Arg Tyr Asn Gln Lys Phe
    50                  55                  60

Lys Asp Lys Ala Ile Leu Thr Val Asp Lys Tyr Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Lys Gly Val Tyr Asp Gly His Trp Phe Phe Asp Val Trp Gly Ala
            100                 105                 110

Gly Thr Ser Val Thr Val Ser Ser
        115                 120

<210> 2
<211> 108
<212> PRT
<213> Homo sapiens

<400> 2
Asp Ile Val Met Thr Gln Ser His Lys Phe Met Ser Thr Ser Val Gly

1               5                   10                  15

Asp Arg Val Ser Ile Thr Cys Lys Ala Ser Gln Asp Val Ser Thr Ala

            20                  25                  30

Val Ala Trp Tyr Gln Gln Lys Pro Gly Gln Ser Pro Lys Leu Leu Ile

        35                  40                  45

Tyr Ser Ala Ser Tyr Arg Tyr Thr Gly Val Pro Ala Arg Phe Ser Gly

    50                  55                  60

Ser Gly Ser Gly Thr Asp Phe Thr Phe Thr Ile Ser Ser Val Gln Thr

65                  70                  75                  80

Glu Asp Leu Ala Val Tyr Tyr Cys Gln Gln His Tyr Arg Thr Pro Pro

                85                  90                  95

Thr Phe Gly Gly Gly Thr Lys Leu Glu Leu Lys Arg

            100                 105

<210>  3

<211>  119

<212>  PRT

<213>  Homo sapiens

<400>  3

Gln Val Gln Leu Gln Gln Ser Gly Pro Glu Leu Glu Lys Pro Gly Ala
1               5                   10                  15

Ser Val Lys Ile Ser Cys Lys Ala Ser Gly Tyr Ser Phe Ser Asp Tyr
            20                  25                  30

Asn Met Asn Trp Val Lys Gln Ser Asn Gly Lys Ser Leu Glu Trp Ile
        35                  40                  45

Gly Asn Ile Asp Pro Tyr Asn Gly Asp Thr Asn Tyr Asn Gln Lys Phe
    50                  55                  60

Lys Gly Lys Ala Thr Leu Thr Leu Asp Lys Ser Ser Ser Thr Ala Tyr
65                  70                  75                  80

Met Gln Leu Lys Ser Leu Thr Ser Glu Asp Ser Ala Val Tyr Phe Cys
                85                  90                  95

Ala Arg Ser Arg Gly Trp Leu Leu Pro Phe Ala Tyr Trp Gly Gln Gly
            100                 105                 110

Thr Leu Val Thr Val Ser Ala
        115

<210>  4

<211>  108

<212>  PRT

<213>  Homo sapiens

<400>  4

Asp Ile Leu Met Thr Gln Ser Gln Lys Phe Met Ser Thr Ser Val Gly
1               5                   10                  15

Asp Arg Val Ser Val Thr Cys Lys Ala Ser Gln Asn Val Gly Ile Asn
            20                  25                  30

Val Ala Trp Tyr Gln Gln Lys Pro Gly Gln Ser Pro Lys Ala Leu Ile
        35                  40                  45

Tyr Ser Ala Ser Tyr Arg Tyr Ser Gly Val Pro Asp Arg Phe Thr Gly
    50                  55                  60

Ser Gly Ser Gly Thr Asp Phe Thr Leu Thr Ile Ser Asn Val Gln Ser
65                  70                  75                  80

Glu Asp Leu Ala Glu Tyr Phe Cys Gln Gln Tyr Asn Ser Tyr Pro Leu
                85                  90                  95

Thr Phe Gly Gly Gly Thr Lys Leu Glu Ile Lys Arg
            100                 105

<210>  5

<211> 117

<212> PRT

<213> Homo sapiens

<400> 5

Gln Val Gln Leu Gln Gln Ser Gly Pro Glu Leu Val Arg Pro Gly Val
1               5                   10                  15

Ser Val Lys Ile Ser Cys Lys Gly Ser Gly Tyr Thr Phe Thr Asp Tyr
            20                  25                  30

Ala Ile His Trp Val Arg Gln Ser His Ala Gln Ser Leu Glu Trp Ile
        35                  40                  45

Gly Val Ile Gly Thr Tyr Ser Gly Asn Arg Asn Tyr Asn Gln Lys Phe
    50                  55                  60

Lys Gly Lys Ala Thr Met Thr Val Asp Lys Ser Ser Ser Thr Ala Tyr
65                  70                  75                  80

Met Glu Leu Ala Arg Leu Thr Ser Glu Asp Ser Ala Ile Tyr Tyr Cys
                85                  90                  95

Ala Arg Ser Ala Gly Tyr Ser Leu Asp Phe Trp Gly Gln Gly Thr Ser
            100                 105                 110

Val Thr Val Ser Ser
        115

<210> 6
<211> 112
<212> PRT
<213> Homo sapiens

<400> 6
Asp Val Val Met Thr Gln Thr Pro Leu Thr Leu Ser Val Thr Ile Gly
1               5                   10                  15

Gln Pro Ala Ser Ile Ser Cys Lys Ser Ser Gln Ser Leu Leu Asp Ser
            20                  25                  30

Asp Gly Lys Thr Tyr Leu Asn Trp Leu Leu Gln Arg Pro Gly Gln Ser
        35                  40                  45

Pro Lys Arg Leu Ile Tyr Leu Val Ser Lys Leu Asp Ser Gly Val Pro
    50                  55                  60

Asp Arg Phe Thr Gly Ser Gly Ser Gly Thr Asp Phe Thr Leu Lys Ile
65                  70                  75                  80

Ser Arg Val Glu Ala Glu Asp Leu Gly Val Tyr Tyr Cys Trp Gln Gly
                85                  90                  95

Lys His Phe Pro Trp Thr Phe Gly Gly Gly Thr Lys Leu Glu Ile Lys
            100                 105                 110

<210> 7
<211> 119
<212> PRT
<213> Homo sapiens

<400> 7
Gln Val Gln Leu Gln Gln Ser Gly Gly Glu Leu Val Arg Pro Gly Thr
1               5                   10                  15

Ser Val Lys Val Ser Cys Lys Ala Ser Gly Tyr Ala Phe Thr Asn Tyr
            20                  25                  30

Leu Ile Glu Trp Ile Arg Gln Arg Pro Gly Gln Gly Leu Glu Trp Ile
        35                  40                  45

Gly Val Ile Asn Pro Gly Ser Gly Asn Ser Lys Ser Ser Lys Asn Leu
    50                  55                  60

Lys Gly Lys Ala Thr Leu Thr Ala Asp Lys Ser Ser Asn Thr Ala Tyr
65                  70                  75                  80

Met Gln Leu Ser Ser Leu Thr Ser Asp Asp Ser Ala Val Tyr Phe Cys

                85                  90                  95

Ala Arg Ser Gly Val Tyr Gly Ser Ser Pro Asp Tyr Trp Gly Gln Gly

            100                 105                 110

Thr Thr Leu Thr Val Ser Ser

        115

<210> 8

<211> 113

<212> PRT

<213> Homo sapiens

<400> 8

Asp Val Val Met Thr Gln Thr Pro Leu Thr Leu Ser Val Thr Ile Gly

1               5                   10                  15

Gln Pro Ala Ser Ile Ser Cys Lys Ser Ser Gln Ser Leu Leu Asp Ser

            20                  25                  30

Asp Gly Lys Thr Tyr Leu Asn Trp Leu Leu Gln Arg Pro Gly Gln Ser

        35                  40                  45

Pro Lys Arg Leu Ile Tyr Leu Val Ser Lys Leu Asp Ser Gly Val Pro

    50                  55                  60

Asp Arg Phe Thr Gly Ser Gly Ser Gly Thr Asp Phe Thr Leu Lys Ile
65                  70                  75                  80

Ser Arg Val Glu Ala Glu Asp Leu Gly Val Tyr Tyr Cys Trp Gln Gly
                85                  90                  95

Thr His Phe Pro Gln Thr Phe Gly Gly Gly Thr Lys Leu Glu Ile Lys
            100                 105                 110

Arg

<210>  9
<211>  118
<212>  PRT
<213>  Homo sapiens

<400>  9
Gln Val Gln Leu Gln Gln Ser Gly Gly Glu Leu Val Arg Pro Gly Thr
1               5                   10                  15

Ser Val Lys Val Ser Cys Lys Ala Ser Gly Tyr Ala Phe Thr Asn Tyr
            20                  25                  30

Leu Ile Glu Trp Val Lys Gln Arg Pro Gly Gln Gly Leu Asp Trp Ile
        35                  40                  45

Gly Met Ile Asn Pro Gly Ser Gly Gly Thr Lys Cys Asn Lys Lys Phe
    50                  55                  60

Lys Gly Lys Val Thr Leu Thr Ala Asp Lys Ser Ser Ser Thr Ala Tyr
65                  70                  75                  80

Met His Leu Ser Ser Leu Thr Ser Asp Asp Ser Ala Val Tyr Phe Cys
                85                  90                  95

Ala Arg Ser Gly Trp Val Ser Ala Met Asp Tyr Trp Gly Gln Gly Thr
            100                 105                 110

Ser Val Thr Val Ser Ser
        115

<210>  10
<211>  113
<212>  PRT
<213>  Homo sapiens

<400>  10
Asp Ile Val Met Thr Gln Thr Pro Leu Thr Leu Ser Val Thr Ile Gly
1               5                   10                  15

Gln Pro Ala Ser Ile Ser Cys Lys Ser Ser Gln Ser Leu Leu Asp Ser

            20                  25                  30

Asp Gly Lys Thr Tyr Leu Asn Trp Leu Leu Gln Arg Pro Gly Gln Ser

        35                  40                  45

Pro Lys Arg Leu Ile Tyr Leu Val Ser Lys Leu Asp Ser Gly Val Pro

    50                  55                  60

Asp Arg Phe Thr Gly Ser Gly Ser Gly Thr Asp Phe Thr Leu Lys Ile

65                  70                  75                  80

Ser Arg Val Glu Ala Glu Asp Leu Gly Val Tyr Tyr Cys Trp Gln Gly

                85                  90                  95

Thr His Phe Pro Gln Thr Phe Gly Gly Gly Thr Lys Leu Glu Leu Lys

            100                 105                 110

Arg

<210> 11

<211> 118

<212> PRT

<213> Homo sapiens

<400> 11

Gln Val Gln Leu Gln Gln Ser Gly Val Glu Leu Val Arg Pro Gly Thr
1               5                   10                  15

Ser Val Lys Val Ser Cys Lys Ala Ser Gly Tyr Ala Phe Thr Asn Tyr
            20                  25                  30

Leu Ile Glu Trp Val Lys Gln Arg Pro Gly Gln Gly Leu Asp Trp Ile
        35                  40                  45

Gly Met Ile Asn Pro Gly Ser Gly Gly Thr Lys Cys Asn Lys Lys Phe
    50                  55                  60

Lys Gly Lys Val Thr Leu Thr Ala Asp Lys Ser Ser Ser Thr Ala Tyr
65                  70                  75                  80

Met His Leu Ser Ser Leu Thr Ser Asp Asp Ser Ala Val Tyr Phe Cys
                85                  90                  95

Ala Arg Ser Gly Trp Val Tyr Ala Met Asp Tyr Trp Gly Gln Gly Thr
            100                 105                 110

Ser Val Thr Val Ser Ser
        115

<210>  12

<211>  113

<212>  PRT

<213>  Homo sapiens

<400>  12

Asp Val Leu Met Thr Gln Thr Pro Leu Thr Leu Ser Val Thr Ile Gly
1               5                   10                  15

Gln Pro Ala Ser Ile Ser Cys Lys Ser Ser Gln Ser Leu Leu Asp Ser
            20                  25                  30

Asp Gly Lys Thr Tyr Leu Asn Trp Leu Leu Gln Arg Pro Gly Gln Ser
        35                  40                  45

Pro Lys Arg Leu Ile Tyr Leu Val Ser Lys Leu Asp Ser Gly Val Pro
    50                  55                  60

Asp Arg Phe Thr Gly Ser Gly Ser Gly Thr Asp Phe Thr Leu Lys Ile
65                  70                  75                  80

Ser Arg Val Glu Ala Glu Asp Leu Gly Val Tyr Tyr Cys Trp Gln Gly
                85                  90                  95

Thr His Phe Pro Gln Thr Phe Gly Gly Gly Thr Lys Leu Glu Leu Lys
            100                 105                 110

Arg

<210> 13

<211> 117

<212> PRT

<213> Homo sapiens

<400> 13

Gln Val Gln Leu Gln Gln Ser Gly Pro Glu Leu Val Arg Pro Gly Val

1               5                   10                  15

Ser Val Lys Ile Ser Cys Lys Gly Ser Gly Tyr Arg Phe Thr Asp Tyr

            20                  25                  30

Ala Ile His Trp Val Lys Gln Ser His Ala Lys Ser Leu Glu Trp Ile

        35                  40                  45

Gly Val Ile Ser Thr Tyr Tyr Gly Asn Thr Arg Tyr Asn Gln Lys Phe

    50                  55                  60

Lys Gly Lys Ala Thr Met Thr Val Asp Lys Ser Ser Ser Thr Ala Tyr

65                  70                  75                  80

Met Glu Leu Ala Ser Leu Thr Ser Glu Asp Ser Val Ile Tyr Tyr Cys

                85                  90                  95

Ala Arg Ser Gly Gly Ser Leu Met Asp Tyr Trp Gly Gln Gly Thr Ser
            100                 105                 110

Val Thr Val Ser Ser
        115

<210> 14
<211> 113
<212> PRT
<213> Homo sapiens

<400> 14
Asp Ile Val Met Thr Gln Thr Pro Leu Thr Leu Ser Val Thr Ile Gly
1               5                   10                  15

Gln Pro Ala Ser Ile Ser Cys Lys Ser Ser Gln Ser Leu Leu Asp Ser
            20                  25                  30

Asp Gly Lys Thr Tyr Leu Asn Trp Leu Leu Gln Arg Pro Gly Gln Ser
        35                  40                  45

Pro Lys Arg Leu Ile Tyr Leu Val Ser Lys Leu Asp Ser Gly Val Pro
    50                  55                  60

Asp Arg Phe Thr Gly Ser Gly Ser Gly Thr Asp Phe Thr Leu Lys Ile
65                  70                  75                  80

Ser Arg Val Glu Ala Glu Asp Leu Gly Val Tyr Tyr Cys Trp Gln Gly
                85                  90                  95

Thr His Phe Pro Tyr Thr Phe Gly Gly Gly Thr Lys Leu Glu Ile Lys
            100                 105                 110

Arg

<210>  15
<211>  117
<212>  PRT
<213>  Homo sapiens

<400>  15
Gln Val Gln Leu Gln Gln Ser Gly Pro Glu Leu Val Arg Pro Gly Val
1               5                   10                  15

Ser Val Lys Ile Ser Cys Lys Gly Ser Gly Tyr Thr Phe Thr Asp Tyr
            20                  25                  30

Ala Met His Trp Val Lys Gln Ser His Ala Lys Ser Leu Glu Trp Ile
        35                  40                  45

Gly Val Ile Ser Thr Tyr Tyr Ser Asn Thr Arg Tyr Asn Gln Lys Phe

    50                  55                  60

Lys Gly Lys Ala Thr Met Thr Val Asp Lys Ser Ser Ser Thr Ala Tyr
65                  70                  75                  80

Met Glu Leu Ala Arg Leu Thr Ser Glu Asp Ser Ala Ile Tyr Tyr Cys
                85                  90                  95

Val Arg Ser Gly Gly Ser Asn Met Asp Tyr Trp Gly Gln Gly Thr Ser
            100                 105                 110

Val Thr Val Ser Ser
        115

<210> 16
<211> 113
<212> PRT
<213> Homo sapiens

<400> 16
Asp Ile Gln Met Thr Gln Thr Pro Leu Thr Leu Ser Val Thr Ile Gly
1               5                   10                  15

Gln Pro Ala Ser Ile Ser Cys Lys Ser Ser Gln Ser Leu Leu Asp Ser
            20                  25                  30

Asp Gly Lys Thr Tyr Leu Asn Trp Leu Leu Gln Arg Pro Gly Gln Ser
        35                  40                  45

Pro Lys Arg Leu Ile Tyr Leu Val Ser Lys Leu Asp Ser Gly Val Pro
    50                  55                  60

Asp Arg Phe Thr Gly Ser Gly Ser Gly Thr Asp Phe Thr Leu Lys Ile
65                  70                  75                  80

Ser Arg Val Glu Ala Glu Asp Leu Gly Val Tyr Tyr Cys Trp Gln Gly
                85                  90                  95

Thr His Phe Pro Trp Thr Phe Gly Gly Gly Thr Lys Leu Glu Ile Lys
            100                 105                 110

Arg

<210>  17
<211>  117
<212>  PRT
<213>  Homo sapiens

<400>  17
Gln Val Gln Leu Gln Gln Ser Gly Pro Glu Leu Val Arg Pro Gly Val
1               5                   10                  15

Ser Val Lys Ile Ser Cys Lys Gly Ser Ser Tyr Lys Phe Thr Asp Tyr
            20                  25                  30
Ala Met His Trp Val Lys Gln Ser His Ala Lys Ser Leu Glu Trp Ile
        35                  40                  45
Gly Val Ile Ser Thr Tyr Tyr Gly Asn Val Lys Tyr Asn Gln Lys Phe
    50                  55                  60
Lys Gly Lys Ala Thr Met Thr Val Asp Lys Ser Ser Ser Thr Ala Tyr
65                  70                  75                  80
Met Glu Leu Ala Arg Leu Thr Ser Glu Asp Ser Ala Val Tyr Tyr Cys
                85                  90                  95
Ala Arg Ser Ser Gly Ser Tyr Leu Asp Tyr Trp Gly Gln Gly Thr Ser
            100                 105                 110
Val Thr Val Ser Ser
        115
<210> 18
<211> 113
<212> PRT
<213> Homo sapiens

<400> 18

Asp Ile Val Met Thr Gln Thr Pro Leu Thr Leu Ser Val Thr Ile Gly

1               5                   10                  15

Gln Pro Ala Ser Ile Ser Cys Lys Ser Ser Gln Ser Leu Leu Asp Ser

            20                  25                  30

Asp Gly Lys Thr Tyr Leu Asn Trp Leu Leu Gln Arg Pro Gly Gln Ser

        35                  40                  45

Pro Lys Arg Leu Ile Tyr Leu Val Ser Lys Leu Asp Ser Gly Val Pro

    50                  55                  60

Asp Arg Phe Thr Gly Ser Gly Ser Gly Thr Asp Phe Thr Leu Lys Ile

65                  70                  75                  80

Ser Arg Val Glu Ala Glu Asp Leu Gly Val Tyr Tyr Cys Trp Gln Gly

                85                  90                  95

Thr His Phe Pro Tyr Thr Phe Gly Gly Gly Thr Lys Leu Glu Ile Lys

            100                 105                 110

Arg

<210>  19

<211>  119

<212>  PRT

<213>  Homo sapiens

<400>  19

Gln Val Gln Leu Gln Gln Ser Gly Ala Glu Leu Val Arg Pro Gly Thr
1               5                   10                  15

Ser Val Lys Val Ser Cys Lys Ala Ser Gly Tyr Ala Phe Thr Asn Tyr
            20                  25                  30

Leu Ile Glu Trp Val Lys Gln Arg Pro Gly Gln Gly Pro Glu Trp Ile
        35                  40                  45

Gly Val Ile Asn Pro Gly Ser Gly Asn Ile Arg Tyr Asn Gly Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Asp Asp Ser Ala Val Tyr Phe Cys
                85                  90                  95

Ala Arg Asp Ala Tyr Tyr Val Gly Ala Met Asp Tyr Trp Gly Gln Gly
            100                 105                 110

Thr Ser Val Thr Val Ser Ser
        115

<210> 20
<211> 113
<212> PRT
<213> Homo sapiens

<400> 20
Asp Val Val Met Thr Gln Thr Pro Leu Thr Leu Ser Val Thr Ile Gly
1               5                   10                  15

Gln Pro Ala Ser Ile Ser Cys Lys Ser Ser Gln Ser Leu Leu Asp Ser
            20                  25                  30

Asp Gly Lys Thr Tyr Leu Asn Trp Leu Leu Gln Arg Pro Gly Gln Ser
        35                  40                  45

Pro Lys Arg Leu Ile Tyr Leu Val Ser Lys Leu Asp Ser Gly Val Pro
    50                  55                  60

Asp Arg Phe Thr Gly Ser Gly Ser Gly Thr Asp Phe Thr Leu Lys Ile
65                  70                  75                  80

Ser Arg Val Glu Ala Glu Asp Leu Gly Val Tyr Tyr Cys Trp Gln Gly

                85                  90                  95

Thr His Phe Pro Gln Thr Phe Gly Gly Gly Thr Lys Leu Glu Leu Lys

            100                 105                 110

Arg

<210> 21

<211> 119

<212> PRT

<213> Homo sapiens

<400> 21

Gln Val Gln Leu Gln Gln Ser Glu Ala Glu Leu Val Arg Pro Glu Thr

1               5                   10                  15

Ser Val Lys Val Ser Cys Lys Ala Ser Gly Tyr Ser Phe Arg Asn Tyr

            20                  25                  30

Leu Ile Glu Trp Val Lys Gln Arg Pro Gly Gln Gly Leu Glu Trp Ile

        35                  40                  45

Gly Val Ile Asn Pro Gly Ser Gly Asn Thr Lys Tyr Asn Glu Lys Phe

    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Asp Asp Ser Ala Val Tyr Phe Cys
                85                  90                  95

Ala Arg Asp Gly Tyr Tyr Leu Gly Thr Met Asp Tyr Trp Gly Gln Gly
            100                 105                 110

Thr Ser Val Thr Val Ser Ser
        115

<210>  22
<211>  113
<212>  PRT
<213>  Homo sapiens

<400>  22
Asp Ile Val Leu Thr Gln Thr Pro Leu Thr Leu Ser Val Thr Ile Gly
1               5                   10                  15

Gln Pro Ala Ser Ile Ser Cys Lys Ser Ser Gln Ser Leu Leu Asp Ser
            20                  25                  30

Asp Gly Lys Thr Tyr Leu Asn Trp Leu Leu Gln Arg Pro Gly Gln Ser
        35                  40                  45

Pro Lys Arg Leu Ile Tyr Leu Val Ser Lys Leu Asp Ser Gly Val Pro
    50                  55                  60

Asp Arg Phe Thr Gly Ser Gly Ser Gly Thr Asp Phe Thr Leu Lys Ile
65                  70                  75                  80

Ser Arg Val Glu Ala Glu Asp Leu Gly Val Tyr Tyr Cys Trp Gln Gly
                85                  90                  95

Thr His Phe Pro Tyr Thr Phe Gly Gly Gly Thr Lys Leu Glu Ile Lys
            100                 105                 110

Arg

<210> 23
<211> 119
<212> PRT
<213> Homo sapiens

<400> 23
Gln Val Gln Leu Gln Gln Ser Gly Ala Glu Leu Val Arg Pro Gly Thr
1               5                   10                  15

Ser Val Lys Val Ser Cys Lys Ala Ser Gly Tyr Ala Phe Ile Asn Asn

20                  25                  30

Leu Ile Glu Trp Val Gln Gln Arg Pro Gly Gln Gly Leu Glu Trp Ile

        35                  40                  45

Gly Val Ile Asn Pro Gly Ser Gly Asn Val Lys Tyr Asn Glu Lys Phe

    50                  55                  60

Lys Gly Lys Ala Thr Leu Thr Ala Asp Lys Ser Ser Ser Thr Ala Tyr

65                  70                  75                  80

Met Gln Leu Ser Ser Leu Thr Ser Asp Asp Ser Ala Val Tyr Phe Cys

                85                  90                  95

Ala Arg Asp Gly Tyr Tyr Leu Gly Thr Met Asp His Trp Gly Gln Gly

            100                 105                 110

Thr Ser Val Thr Val Ser Ser

        115

<210>  24

<211>  113

<212>  PRT

<213>  Homo sapiens

<400>  24

Asp Val Val Met Thr Gln Thr Pro Leu Thr Leu Ser Val Thr Ile Gly
1               5                   10                  15

Gln Pro Ala Ser Ile Ser Cys Lys Ser Ser Gln Ser Leu Leu Asp Ser
            20                  25                  30

Asp Gly Lys Thr Tyr Leu Asn Trp Leu Leu Gln Arg Pro Gly Gln Ser
        35                  40                  45

Pro Lys Arg Leu Ile Tyr Leu Val Ser Lys Leu Asp Ser Gly Val Pro
    50                  55                  60

Asp Arg Phe Thr Gly Ser Gly Ser Gly Thr Asp Phe Thr Leu Lys Ile
65                  70                  75                  80

Ser Arg Val Glu Ala Glu Asp Leu Gly Ile Tyr Tyr Cys Trp Gln Gly
                85                  90                  95

Thr His Phe Pro Trp Thr Phe Gly Gly Gly Thr Lys Leu Glu Leu Lys
            100                 105                 110

Arg

<210>  25
<211>  117

<212>  PRT

<213>  Homo sapiens

<400>  25

Glu Val Gln Leu Gln Gln Ser Gly Pro Glu Leu Val Arg Pro Gly Val
1               5                   10                  15

Ser Val Lys Ile Ser Cys Lys Gly Ser Ser Tyr Lys Phe Thr Asp Tyr
            20                  25                  30

Ala Met His Trp Val Lys Gln Ser His Ala Lys Ser Leu Glu Trp Ile
        35                  40                  45

Gly Val Ile Ser Thr Tyr Tyr Gly Asn Val Lys Tyr Asn Gln Lys Phe
    50                  55                  60

Lys Gly Lys Ala Thr Met Thr Val Asp Lys Ser Ser Ser Thr Ala Tyr
65                  70                  75                  80

Met Glu Leu Ala Arg Leu Thr Ser Glu Asp Ser Ala Val Tyr Tyr Cys
                85                  90                  95

Ala Arg Ser Tyr Gly Ser Tyr Leu Asp Tyr Trp Gly Gln Gly Thr Ser
            100                 105                 110

Val Thr Val Ser Ser

        115

<210> 26

<211> 112

<212> PRT

<213> Homo sapiens

<400> 26

Asp Ile Val Met Thr Gln Thr Pro Leu Thr Leu Ser Val Thr Ile Gly

1               5                   10                  15

Gln Pro Ala Ser Ile Ser Cys Lys Ser Ser Gln Ser Leu Leu Asp Ser

            20                  25                  30

Asp Gly Lys Thr Tyr Leu Asn Trp Leu Leu Gln Arg Pro Gly Gln Ser

        35                  40                  45

Pro Lys Arg Leu Ile Tyr Leu Val Ser Lys Leu Asp Ser Gly Val Pro

    50                  55                  60

Asp Arg Phe Thr Gly Ser Gly Ser Gly Thr Asp Phe Thr Leu Lys Ile

65                  70                  75                  80

Ser Arg Val Glu Ala Glu Asp Leu Gly Val Tyr Tyr Cys Trp Gln Gly

                85                  90                  95

Thr His Phe Pro Trp Thr Phe Gly Gly Gly Thr Lys Leu Glu Ile Lys

            100                 105                 110

<210> 27

<211> 22

<212> DNA

<213> Artificial

<220>

<223> an artificially synthesized primer sequence

<400> 27

cagctatgaa atacctattg cc 22

<210> 28

<211> 23

<212> DNA

<213> Artificial

<220>

<223> an artificially synthesized primer sequence

<400> 28

cttttcataa tcaaaatcac cgg 23

<210> 29

<211>  19

<212>  DNA

<213>  Artificial

<220>

<223>  an artificially synthesized primer sequence

<400>  29
attgcctacg gcagccgct                                                    19

<210>  30

<211>  20

<212>  DNA

<213>  Artificial

<220>

<223>  an artificially synthesized primer sequence

<400>  30
aaatcaccgg aaccagagcc                                                   20

<210>  31

<211>  24

<212>  DNA

<213>  Artificial

<220>

<223>  an artificially synthesized primer sequence

<400>  31
ttactcgcgg cccagccggc catg                                              24

<210>  32
<211>  28
<212>  DNA
<213>  Artificial

<220>

<223>  an artificially synthesized primer sequence

<400>  32
   ggaattcggc ccccgaggcc cactcacg                                           28

<210>  33
<211>  1215
<212>  DNA
<213>  Homo sapiens

<400>  33
ggcctcgggg gccagctttc tggggcaggc caggcctgac cttggctttg gggcagggag      60

ggggctaagg tgaggcaggt ggcgccagcc aggtgcacac ccaatgccca tgagcccaga     120

cactggacgc tgaacctcgc ggacagttaa gaacccaggg gcctctgcgc cctgggccca 180

gctctgtccc acaccgcggt cacatggcac cacctctctt gcagcttcca ccaagggccc 240

atccgtcttc cccctggcgc cctgctccag gagcacctcc gagagcacag ccgccctggg 300

ctgcctggtc aaggactact tccccgaacc ggtgacggtg tcgtggaact caggcgccct 360

gaccagcggc gtgcacacct tcccggctgt cctacagtcc tcaggactct actccctcag 420

cagcgtggtg accgtgccct ccagcagctt gggcacgaag acctacacct gcaacgtaga 480

tcacaagccc agcaacacca aggtggacaa gagagttgag tccaaatatg gtcccccatg 540

cccaccatgc ccagcacctg agttcctggg gggaccatca gtcttcctgt tccccccaaa 600

acccaaggac actctcatga tctcccggac ccctgaggtc acgtgcgtgg tggtggacgt 660

gagccaggaa gaccccgagg tccagttcaa ctggtacgtg gatggcgtgg aggtgcataa 720

tgccaagaca aagccgcggg aggagcagtt caacagcacg taccgtgtgg tcagcgtcct 780

caccgtcctg caccaggact ggctgaacgg caaggagtac aagtgcaagg tctccaacaa 840

aggcctcccg tcctccatcg agaaaaccat ctccaaagcc aaagggcagc cccgagagcc 900

acaggtgtgc accctgcccc catcccagga ggagatgacc aagaaccagg tcagcctgtg 960

gtgcctggtc aaaggcttct accccagcga catcgccgtg gagtgggaga gcaatgggca 1020

gccggagaac aactacaaga ccacgcctcc cgtgctggac tccgacggct ccttcttcct 1080

ctacagcagg ctaaccgtgg acaagagcag gtggcaggag gggaatgtct tctcatgctc 1140

cgtgatgcat gaggctctgc acaaccacta cacacagaag agcctctccc tgtctctggg 1200

taaatgagcg gccgc 1215

<210> 34

<211> 684

<212> DNA

<213> Homo sapiens

<400> 34

ggcctcgggg gccgaattcc taaactctga gggggtcgga tgacgtggcc attctttgcc 60

taaagcattg agtttactgc aaggtcagaa aagcatgcaa agccctcaga atggctgcaa 120

agagctccaa caaaacaatt tagaacttta ttaaggaata gggggaagct aggaagaaac 180

tcaaaacatc aagattttaa atacgcttct tggtctcctt gctataatta tctgggataa 240

gcatgctgtt ttctgtctgt ccctaacatg ccctgtgatt atccgcaaac aacacaccca 300

agggcagaac tttgttactt aaacaccatc ctgtttgctt ctttcctcag gaactgtggc 360

tgcaccatct gtcttcatct tcccgccatc tgatgagcag ttgaaatctg gaactgcctc 420

tgttgtgtgc ctgctgaata acttctatcc cagagaggcc aaagtacagt ggaaggtgga 480

taacgccctc caatcgggta actcccagga gagtgtcaca gagcaggaca gcaaggacag 540

cacctacagc ctcagcagca ccctgacgct gagcaaagca gactacgaga aacacaaagt 600

ctacgcctgc gaagtcaccc atcagggcct gagctcgccc gtcacaaaga gcttcaacag 660

gggagagtgt tagagggcgg ccgc 684

<210> 35

<211> 1215

<212> DNA

<213> Homo sapiens

<400> 35

ggcctcgggg gcctcccagg ctctgggcag gcacaggcta ggtgccccta acccaggccc 60

tgcacacaaa ggggcaggtg ctgggctcag acctgccaag agccatatcc gggaggaccc 120

tgcccctgac ctaagcccac cccaaaggcc aaactctcca ctccctcagc tcggacacct 180

tctctcctcc cagattccag taactcccaa tcttctctct gcagcttcca ccaagggccc 240

atccgtcttc cccctggcgc cctgctccag gagcacctcc gagagcacag ccgccctggg 300

ctgcctggtc aaggactact tccccgaacc ggtgacggtg tcgtggaact caggcgccct 360

gaccagcggc gtgcacacct tcccggctgt cctacagtcc tcaggactct actccctcag 420

cagcgtggtg accgtgccct ccagcagctt gggcacgaag acctacacct gcaacgtaga 480

tcacaagccc agcaacacca aggtggacaa gagagttgag tccaaatatg gtcccccatg 540

cccaccatgc ccagcacctg agttcctggg gggaccatca gtcttcctgt tccccccaaa 600

acccaaggac actctcatga tctcccggac ccctgaggtc acgtgcgtgg tggtggacgt 660

gagccaggaa gaccccgagg tccagttcaa ctggtacgtg gatggcgtgg aggtgcataa 720

tgccaagaca aagccgcggg aggagcagtt caacagcacg taccgtgtgg tcagcgtcct 780

caccgtcctg caccaggact ggctgaacgg caaggagtac aagtgcaagg tctccaacaa 840

aggcctcccg tcctccatcg agaaaaccat ctccaaagcc aaagggcagc cccgagagcc 900

acaggtgtac accctgcccc catcccagtg cgagatgacc aagaaccagg tcagcctgtc 960

ctgcgcggtc aaaggcttct atcccagcga catcgccgtg gagtgggaga gcaatgggca 1020

gccggagaac aactacaaga ccacgcctcc cgtgctggac tccgacggct ccttcttcct 1080

cgtgagcagg ctaaccgtgg acaagagcag gtggcaggag gggaatgtct tctcatgctc 1140

cgtgatgcat gaggctctgc acaaccacta cacacagaag agcctctccc tgtctctggg 1200

taaatgagcg gccgc 1215

<210> 36

<211> 21

<212> DNA

<213> Artificial

<220>

<223> an artificially synthesized primer sequence

<400> 36

cgcaaatggg cggtaggcgt g 21

<210> 37

<211> 18

<212>  DNA

<213>  Artificial

<220>

<223>  an artificially synthesized primer sequence

<400>  37

tagaaggcac agtcgagg                                                    18

<210>  38

<211>  24

<212>  DNA

<213>  Artificial

<220>

<223>  an artificially synthesized primer sequence

<400>  38

ctctgaatac tttcaacaag ttac                                             24

<210>  39

<211>  116

<212>  PRT

<213>  Mus musculus

<400>  39

Met Glu Val Gln Leu Gln Gln Ser Gly Pro Gly Leu Val Lys Pro Thr
1               5                   10                  15

Gln Ser Leu Ser Leu Thr Cys Ser Val Thr Gly Tyr Ser Ile Thr Ser
            20                  25                  30

Gly Tyr Tyr Trp Thr Trp Ile Arg Gln Phe Pro Gly Asn Asn Leu Glu
        35                  40                  45

Trp Ile Gly Tyr Ile Ser Phe Asp Gly Thr Asn Asp Tyr Asn Pro Ser
    50                  55                  60

Leu Lys Asn Arg Ile Ser Ile Thr Arg Asp Thr Ser Glu Asn Gln Phe
65                  70                  75                  80

Phe Leu Lys Leu Asn Ser Val Thr Thr Glu Asp Thr Ala Thr Tyr Tyr
                85                  90                  95

Cys Ala Arg Gly Pro Pro Cys Thr Tyr Trp Gly Gln Gly Thr Leu Val
            100                 105                 110

Thr Val Ser Ala
        115

<210>  40
<211>  6

<212>  PRT

<213>  Mus musculus

<400>  40

Ser Gly Tyr Tyr Trp Thr

1               5

<210>  41

<211>  16

<212>  PRT

<213>  Mus musculus

<400>  41

Tyr Ile Ser Phe Asp Gly Thr Asn Asp Tyr Asn Pro Ser Leu Lys Asn

1               5                   10                  15

<210>  42

<211>  6

<212>  PRT

<213>  Mus musculus

<400>  42

Gly Pro Pro Cys Thr Tyr

1               5

<210>  43

<211>  120

<212>  PRT

<213>  Mus musculus

<400>  43

Met Gln Val Gln Leu Gln Gln Ser Gly Ala Glu Leu Val Arg Pro Gly
1               5                   10                  15

Ala Ser Val Lys Leu Ser Cys Thr Ala Ser Gly Phe Asn Ile Lys Asp
            20                  25                  30

Asp Tyr Val His Trp Val Lys Gln Arg Pro Glu Gln Gly Leu Glu Trp
        35                  40                  45

Ile Gly Arg Ile Asp Pro Ala Asp Gly Lys Thr Lys Tyr Ala Pro Lys
    50                  55                  60

Phe Gln Asp Lys Ala Thr Met Thr Ser Asp Thr Ser Ser Asn Thr Ala
65                  70                  75                  80

Tyr Leu Gln Leu Ser Ser Leu Thr Ser Glu Asp Thr Ala Val Tyr Tyr
                85                  90                  95

Cys Val Arg Trp Arg Ile Tyr Tyr Gly Leu Met Asp Tyr Trp Gly Gln
            100                 105                 110

Gly Thr Ser Val Thr Val Ser Ser
        115                 120

<210> 44
<211> 5
<212> PRT
<213> Mus musculus

<400> 44
Asp Asp Tyr Val His
1               5

<210> 45
<211> 17
<212> PRT
<213> Mus musculus

<400> 45
Arg Ile Asp Pro Ala Asp Gly Lys Thr Lys Tyr Ala Pro Lys Phe Gln
1               5                   10                  15

Asp

<210> 46
<211> 10

<212>  PRT

<213>  Homo sapiens

<400>  46

Trp Arg Ile Tyr Tyr Gly Leu Met Asp Tyr

1               5                   10

<210>  47

<211>  123

<212>  PRT

<213>  Mus musculus

<400>  47

Met Gln Val Gln Leu Gln Gln Ser Gly Pro Glu Leu Val Lys Pro Gly

1               5                   10                  15

Ala Ser Val Lys Met Ser Cys Lys Ala Ser Gly Tyr Thr Phe Thr His

            20                  25                  30

Phe Val Leu His Trp Val Lys Gln Asn Pro Gly Gln Gly Leu Glu Trp

        35                  40                  45

Ile Gly Tyr Ile Ile Pro Tyr Asn Asp Gly Thr Lys Tyr Asn Glu Lys

    50                  55                  60

Phe Lys Gly Lys Ala Thr Leu Thr Ser Asp Lys Ser Ser Ser Thr Ala

65                  70                  75                  80

Tyr Met Glu Leu Ser Ser Leu Thr Ser Glu Asp Ser Ala Val Tyr Tyr

                85                  90                  95

Cys Ala Arg Gly Asn Arg Tyr Asp Val Gly Ser Tyr Ala Met Asp Tyr

            100                 105                 110

Trp Gly Gln Gly Thr Ser Val Thr Val Ser Ser

        115                 120

<210>  48

<211>  5

<212>  PRT

<213>  Mus musculus

<400>  48

His Phe Val Leu His

1               5

<210>  49

<211>  17

<212>  PRT

<213>  Mus musculus

<400>  49

Tyr Ile Ile Pro Tyr Asn Asp Gly Thr Lys Tyr Asn Glu Lys Phe Lys
1               5                   10                  15

Gly

<210>  50
<211>  13
<212>  PRT
<213>  Mus musculus

<400>  50
Gly Asn Arg Tyr Asp Val Gly Ser Tyr Ala Met Asp Tyr
1               5                   10

<210>  51
<211>  117
<212>  PRT
<213>  Mus musculus

<400>  51
Met Gln Val Gln Leu Gln Gln Ser Gly Ala Glu Leu Val Lys Pro Gly
1               5                   10                  15

Ala Ser Val Lys Leu Ser Cys Thr Val Ser Gly Phe Asn Ile Gln Asp
            20                  25                  30

Asn Tyr Met His Trp Val Lys Gln Arg Pro Glu Gln Gly Leu Glu Trp
        35                  40                  45
Ile Gly Arg Ile Asp Pro Ala Asn Gly Asn Thr Arg Tyr Asp Pro Lys
    50                  55                  60
Phe Gln Gly Lys Ala Thr Ile Thr Ala Asp Ile Ser Ser Asn Thr Thr
65                  70                  75                  80
Cys Leu Gln Leu Ser Ser Leu Thr Ser Glu Asp Thr Ala Val Tyr Tyr
                85                  90                  95
Cys Ala Ser Pro Tyr Tyr Pro Leu Gly Cys Trp Gly Gln Gly Thr Leu
            100                 105                 110
Val Thr Val Ser Ala
        115
<210> 52
<211> 5
<212> PRT
<213> Mus musculus
<400> 52
Asp Asn Tyr Met His

1                   5

<210>  53

<211>  17

<212>  PRT

<213>  Mus musculus

<400>  53

Arg Ile Asp Pro Ala Asn Gly Asn Thr Arg Tyr Asp Pro Lys Phe Gln

1                   5                   10                  15

Gly

<210>  54

<211>  7

<212>  PRT

<213>  Mus musculus

<400>  54

Pro Tyr Tyr Pro Leu Gly Cys

1                   5

<210>  55

<211>  116

<212>  PRT

<213>  Mus musculus

<400>  55

Met Gln Val Gln Leu Gln Gln Ser Gly Pro Glu Leu Val Lys Pro Gly
1               5                   10                  15

Ala Ser Val Lys Ile Ser Cys Lys Thr Ser Gly Tyr Thr Phe Thr Glu
            20                  25                  30

Asn Thr Ile Tyr Trp Val Lys Gln Ser His Gly Lys Ser Leu Glu Trp
        35                  40                  45

Ile Gly Ser Ile Thr Thr Tyr Asn Gln Lys Phe Lys Asp Lys Ala Thr
    50                  55                  60

Leu Thr Ile Asp Lys Ser Ser Ser Ser Ala Tyr Met Glu Leu Arg Ser
65                  70                  75                  80

Leu Thr Ser Glu Glu Ser Ala Val Tyr Tyr Cys Ala Arg Ser Gly Gly
                85                  90                  95

Arg Gly Lys Pro Tyr Tyr Phe Asp Ser Trp Gly Gln Gly Thr Thr Leu
            100                 105                 110

Thr Val Ser Ser
        115

<210> 56

<211> 5

<212> PRT

<213> Mus musculus

<400> 56

Glu Asn Thr Ile Tyr

1               5

<210> 57

<211> 11

<212> PRT

<213> Mus musculus

<400> 57

Ser Ile Thr Thr Tyr Asn Gln Lys Phe Lys Asp

1               5                   10

<210> 58

<211> 12

<212> PRT

<213> Mus musculus

<400> 58

Ser Gly Gly Arg Gly Lys Pro Tyr Tyr Phe Asp Ser

1                5                   10

<210>  59

<211>  117

<212>  PRT

<213>  Mus musculus

<400>  59

Met Gln Val Gln Leu Gln Gln Ser Gly Ser Glu Leu Val Lys Pro Gly

1               5                   10                  15

Ala Ser Val Lys Leu Ser Cys Thr Ala Ser Gly Phe Asn Ile Lys Asp

            20                  25                  30

Asn Tyr Met His Trp Ile Lys Gln Arg Pro Glu Gln Gly Leu Glu Trp

        35                  40                  45

Ile Gly Arg Ile Asp Pro Gly Asn Gly Asn Ser Arg Tyr Asp Pro Lys

    50                  55                  60

Phe Gln Gly Lys Ala Thr Ile Thr Ala Asp Thr Ser Ser Asn Thr Ala

65                  70                  75                  80

Tyr Leu Gln Leu Ser Ser Leu Thr Ser Glu Asp Thr Ala Val Tyr Tyr

                85                  90                  95

Cys Ala Ser Pro Tyr Tyr Pro Leu Gly Tyr Trp Gly Gln Gly Thr Leu
            100                 105                 110

Val Thr Val Ser Ala
        115

<210> 60
<211> 5
<212> PRT
<213> Mus musculus

<400> 60
Asp Asn Tyr Met His
1               5

<210> 61
<211> 17
<212> PRT
<213> Mus musculus

<400> 61
Arg Ile Asp Pro Gly Asn Gly Asn Ser Arg Tyr Asp Pro Lys Phe Gln
1               5                   10                  15

Gly

<210> 62

<211> 7

<212> PRT

<213> Mus musculus

<400> 62

Pro Tyr Tyr Pro Leu Gly Tyr

1               5

<210> 63

<211> 114

<212> PRT

<213> Mus musculus

<400> 63

Met Gln Val Gln Leu Gln Gln Ser Gly Ala Glu Leu Val Arg Pro Gly

1               5                   10                  15

Ala Ser Val Lys Leu Ser Cys Thr Val Ser Gly Phe Asn Ile Lys Asp

            20                  25                  30

Asp Tyr Ile His Trp Val Lys Gln Arg Pro Glu Gln Gly Leu Glu Trp

        35                  40                  45

Ile Gly Arg Ile Asp Pro Thr Asn Gly Asn Pro Ala Tyr Ala Pro Lys

    50                  55                  60

Phe Gln Asp Lys Ala Thr Ile Thr Ala Asp Thr Ser Ser Ile Thr Ala
65                  70                  75                  80

Tyr Leu Gln Leu Asn Ser Leu Thr Ser Glu Asp Thr Ala Val Tyr Tyr
                85                  90                  95

Cys Thr Gly Ser Phe Ala Tyr Trp Gly Gln Gly Thr Leu Val Thr Val
            100                 105                 110

Ser Ala

<210> 64
<211> 5
<212> PRT
<213> Mus musculus

<400> 64
Asp Asp Tyr Ile His
1               5

<210> 65
<211> 17
<212> PRT

<213>  Mus musculus

<400>  65

Arg Ile Asp Pro Thr Asn Gly Asn Pro Ala Tyr Ala Pro Lys Phe Gln
1               5                   10                  15

Asp

<210>  66
<211>  4
<212>  PRT
<213>  Mus musculus

<400>  66
Ser Phe Ala Tyr
1

<210>  67
<211>  114
<212>  PRT
<213>  Mus musculus

<400>  67
Met Gln Val Gln Leu Gln Gln Ser Gly Ala Glu Leu Val Arg Pro Gly
1               5                   10                  15

Ala Ser Val Lys Leu Ser Cys Thr Ala Ser Gly Phe Asn Ile Lys Asp
            20                  25                  30
Asp Tyr Val His Trp Val Lys Gln Arg Pro Glu Gln Gly Leu Glu Trp
        35                  40                  45
Ile Gly Arg Ile His Pro Ala Asn Gly Asn Pro Gln Tyr Ala Pro Lys
    50                  55                  60
Phe Gln Asp Lys Ala Thr Ile Ile Ile Gly Thr Ala Ser Asn Thr Thr
65                  70                  75                  80
Tyr Leu Gln Leu Ser Ser Leu Thr Ser Glu Asp Thr Ala Val Tyr Tyr
                85                  90                  95
Cys Ala Gly Pro Phe Ala Tyr Trp Gly Gln Gly Thr Leu Val Thr Val
            100                 105                 110
Ser Ala
<210> 68
<211> 5
<212> PRT
<213> Mus musculus

<400>  68

Asp Asp Tyr Val His
1               5

<210>  69

<211>  17

<212>  PRT

<213>  Mus musculus

<400>  69

Arg Ile His Pro Ala Asn Gly Asn Pro Gln Tyr Ala Pro Lys Phe Gln
1               5                   10                  15

Asp

<210>  70

<211>  4

<212>  PRT

<213>  Mus musculus

<400>  70

Pro Phe Ala Tyr
1

<210> 71

<211> 116

<212> PRT

<213> Mus musculus

<400> 71

Met Glu Val Gln Leu Gln Glu Ser Gly Pro Gly Leu Val Lys Pro Ser

1               5                   10                  15

Gln Ser Leu Ser Leu Thr Cys Ser Val Thr Gly Tyr Ser Ile Thr Ser

            20                  25                  30

Asn Tyr Tyr Trp Asn Trp Ile Arg Gln Phe Pro Gly Asn Lys Leu Glu

        35                  40                  45

Trp Met Gly Tyr Ile Asn Tyr Asp Gly Ser Asn Asn Tyr Asn Pro Ser

    50                  55                  60

Leu Lys Asn Arg Ile Ser Ile Ser Arg Asp Thr Ser Lys Asn Gln Phe

65                  70                  75                  80

Phe Leu Lys Leu Asn Ser Val Thr Thr Glu Asp Thr Ala Thr Tyr Tyr

                85                  90                  95

Cys Ala Arg Gly Gly Ala Phe Thr Tyr Trp Gly Gln Gly Thr Leu Val

            100                 105                 110

Thr Val Ser Ala

        115

<210>  72

<211>  6

<212>  PRT

<213>  Mus musculus

<400>  72

Ser Asn Tyr Tyr Trp Asn

1               5

<210>  73

<211>  16

<212>  PRT

<213>  Mus musculus

<400>  73

Tyr Ile Asn Tyr Asp Gly Ser Asn Asn Tyr Asn Pro Ser Leu Lys Asn

1               5                   10                  15

<210>  74

<211>  6

<212>  PRT

<213>  Mus musculus

<400> 74

Gly Gly Ala Phe Thr Tyr

1               5

<210> 75

<211> 114

<212> PRT

<213> Mus musculus

<400> 75

Met Gln Val Gln Leu Gln Gln Ser Gly Pro Glu Leu Val Lys Pro Gly

1               5                   10                  15

Ala Ser Val Lys Ile Ser Cys Lys Ala Ser Gly Tyr Thr Ile Thr Asp

            20                  25                  30

Asn Lys Met Asp Trp Val Lys Gln Ser His Gly Lys Ser Leu Glu Trp

        35                  40                  45

Ile Gly Tyr Ile Ser Pro Asn Asn Gly Asp Ile Gly Tyr Asn Arg Lys

    50                  55                  60

Phe Arg Asn Lys Ala Thr Leu Thr Val Asp Lys Ser Ser Ser Thr Ala

65                  70                  75                  80

Tyr Met Glu Leu His Ser Leu Thr Ser Glu Asp Ser Ala Val Tyr Tyr
                85                  90                  95
Cys Ala Arg His Arg Ala Tyr Trp Gly Gln Gly Thr Leu Val Thr Val
            100                 105                 110
Ser Ala
<210> 76
<211> 5
<212> PRT
<213> Mus musculus
<400> 76
Asp Asn Lys Met Asp
1               5
<210> 77
<211> 17
<212> PRT
<213> Mus musculus
<400> 77
Tyr Ile Ser Pro Asn Asn Gly Asp Ile Gly Tyr Asn Arg Lys Phe Arg
1               5                   10                  15

Asn

<210> 78
<211> 4
<212> PRT
<213> Mus musculus

<400> 78
His Arg Ala Tyr
1

<210> 79
<211> 121
<212> PRT
<213> Mus musculus

<400> 79
Met Asp Val Gln Leu Val Glu Ser Gly Gly Gly Leu Val Lys Pro Gly
1               5                   10                  15

Gly Ser Leu Lys Leu Ser Cys Ala Ala Ser Gly Phe Thr Phe Ser Thr
            20                  25                  30

Tyr Ala Met Ser Trp Val Arg Gln Thr Pro Glu Lys Arg Leu Glu Trp

        35                  40                  45

Val Ala Tyr Ile Ser Asn Gly Gly Ala Asn Thr Tyr Tyr Pro Asp Ser

    50                  55                  60

Val Lys Gly Arg Phe Thr Ile Ser Arg Asp Asn Ala Lys Asn Thr Leu

65                  70                  75                  80

Tyr Leu Gln Met Ser Ser Leu Arg Ser Glu Asp Thr Ala Leu Tyr Tyr

                85                  90                  95

Cys Ala Arg Gly Gly Tyr Arg Tyr Pro Tyr Ala Met Asp Tyr Trp Gly

            100                 105                 110

Gln Gly Thr Ser Val Thr Val Ser Ser

        115                 120

<210> 80

<211> 5

<212> PRT

<213> Mus musculus

<400> 80

Thr Tyr Ala Met Ser

1               5

<210> 81

<211> 17

<212> PRT

<213> Mus musculus

<400> 81

Tyr Ile Ser Asn Gly Gly Ala Asn Thr Tyr Tyr Pro Asp Ser Val Lys

1               5                   10                  15

Gly

<210> 82

<211> 11

<212> PRT

<213> Mus musculus

<400> 82

Gly Gly Tyr Arg Tyr Pro Tyr Ala Met Asp Tyr

1               5                   10

## Description

### Technical Field

The present invention relates to bispecific antibodies that substitute for functional proteins. More specifically, the present invention relates to bispecific antibodies that functionally substitute for ligands of hetero-receptors, bispecific antibodies that substitute for the cofactors which enhance enzymatic reaction, and pharmaceutical compositions comprising the antibodies as an active ingredient.

### Background Art

Antibodies have received much attention as a medicine because of their high stability in blood and low antigenicity. Of these are bispecific antibodies that can simultaneously recognize two types of antigens. Bispecific antibodies have been proposed for some time; however, only antibodies that simply connect two types of antigens, such as those for retargeting NK cells, macrophages, and T cells (see Non-Patent Document 8), have been reported. For example, MDX-210, which is currently under clinical study, is a bispecific antibody that merely retargets FcγRI-expressing monocytes and such to HER-2/neu-expressing cancer cells. Thus, so far there are no examples that utilize a bispecific antibody as an alternative means to substitute for an *in vivo* functional protein.

One example of an *in vivo* functional protein is the ligand of a receptor. Examples of such ligands are interleukin (IL)- 2, 3, 4, 5, 6, 7, 9, 10, 11, 12, 13, and 15, erythropoietin (EPO), growth hormone (GH), granulocyte colony-stimulating factor (G-CSF), thrombopoietin (TPO), granulocyte-macrophage colony-stimulating factor (GM-CSF), macrophage colony-stimulating factor (M-CSF), interferon (IFN-α, IFN-β, IFN-γ etc.), ciliary neurotrophic factor (CNTF), leukemia inhibitory factor (LIF), Oncostatin M, Cardiotrophin-1 (CT-1), and tumor necrosis factor (TNF).

In these receptors, it is thought that the distance and/or angle of the receptor molecules forming dimers or multimers change upon ligand binding, thus enabling these receptors to transmit signals into cells. In other words, a suitable anti-receptor antibody may become an antibody that can mimic ligand-mediated receptor dimerization or multimerization.

Monoclonal antibodies that show a ligand-substituting effect towards homodimer-comprising TPO receptors (MPL) (see Patent Document 1 and Non-Patent Document 1), EPO receptors, and GH receptors have already been reported. Respectively, these antibodies are thought to have an effect of recovering thrombocyte count at the time of thrombopenia, an effect of increasing red blood cell count at the time of anemia, and a growth-enhancing effect on dwarfism. Thus, their applications in medicine are expected.

However, in the case of a heterodimer-forming receptor, which requires the formation of a complex of two or several types of receptor molecules, its ligand function cannot be expected to be substituted by general antibodies. The above-mentioned bispecific antibodies which can recognize two types of receptor molecules with their two arms are thought to be suitable for this purpose, however, no reports have been made.

Another example of an *in vivo* functional protein is a cofactor. Examples of cofactors are tissue factor (TF), blood coagulation factor V (F.V), activated blood coagulation factor V (F.Va), blood coagulation factor VIII (F.VIII), activated blood coagulation factor VIII (F.VIIIa), thrombomodulin (TM), protein S (PS), protein Z (PZ), heparin, complement C4b, complement regulatory factor H, membrane cofactor protein (MCP), and complement receptor 1 (CR1).

Of these, F.VIII/F.VIIIa is a cofactor required for sufficient activity expression of F.IXa. Scheiflinger F. *et al.* discovered that a certain anti-F.IX/F.IXa antibody acts to promote the activation of F.X by F.IXa in a chromogenic assay (see Patent Document 2). However, in an assay that examines the ability for coagulation recovery in F.VIII-deficient plasma, the coagulation recovery ability was observed only when F.IXa was added exogenously, but not if this antibody was used alone.

F.VIIIa has been known to interact not only with F.IXa but also with F.X (see Non-Patent Documents 6 and 7). In this respect, the antibody of Scheiflinger F. *et al.* cannot be said to sufficiently substitute for the function of F.VIII/F.VIIIa, and its activity also seems to be insufficient.

Through dedicated research, the present inventors succeeded in producing bispecific antibodies that substitute for the effect of functional proteins, and thereby completed this invention.
(Patent Document 1) U.S. Patent Application No. 98/17364
(Patent Document 2) WO 01/19992
(Patent Document 3) U.S. Patent No. 4, 474,893
(Patent Document 4) EP 404,097
(Patent Document 5) WO 93/11161
(Patent Document 6) Japanese Patent Application No. 2002-112369
(Patent Document 7) Japanese Patent Application No. 2003-012648
(Patent Document 8) Japanese Patent Application Kokai Publication No. (JP-A) H5-304992
(Patent Document 9) JP-A H2-145187
(Patent Document 10) JP-A H5-213775
(Patent Document 11) JP-A H10-165184
(Patent Document 12) JP-A H11-71288
(Patent Document 13) Japanese Patent Kohyo Publication No. (JP-A) 2002-518041
(Patent Document 14) JP-A H11-506310
(Patent Document 15) JP-A H5-199894
(Patent Document 16) JP-A H10-511085
(Patent Document 17) JP-A H5-184383
(Non-Patent Document 1) Deng D *et al.,* "Blood", 1998, Vol.92, No.6, p.1981-1988
(Non-Patent Document 2) Nilsson IM *et al.,* "J. Intern. Med.", 1992 Vol.235, p.25-32
(Non-Patent Document 3) Löfqvist T *et al.,* "J. Intern. Med.", 1997 Vol.241, p.395-400
(Non-Patent Document 4) 24^{th} Meeting of The Japanese Society on Thrombosis and Hematosis, Special Committee on Examining Hemophilia Standardization, Mini-symposium, 2001, http://www.jsth.org
(Non-Patent Document 5) Bulletin #193 1994
(Non-Patent Document 6) Mertens K *et al.,* "Thromb. Haemost.", 1999, Vol.82, p.209-217
(Non-Patent Document 7) Lapan KA *et al.,* "Thromb. Haemost.", 1998, Vol.80, p.418-422
(Non-Patent Document 8) Segal DM *et al.,* "Journal of Immunological Methods", 2001, Vol.248, p. 1-6
(Non-Patent Document 9) Bos R and Nieuwenhuitzen W, "Hybridoma", 1992, Vol. 11, No. 1, p.41-51
(Non-Patent Document 10) Brennan M *et al.,* "Science", 1985, Vol.229, No.1708, p.81-3
(Non-Patent Document 11) Karpovsky B *et al.,* "J. Exp. Med.", 1984, Vol. 160, No.6, p.1686-701
(Non-Patent Document 12) Suresh MR *et al.,* "Methods Enzymol.", 1986, Vol.121, p. 210-28
(Non-Patent Document 13) Massimo YS *et al.,* "J. Immunol. Methods", 1997, Vol.201, p.57-66 (Non-Patent Document 14) Brennan M *et al.,* "Science", 1985, Vol.229, p. 81
(Non-Patent Document 15) Shalaby MR *et al.,* "J. Exp. Med.", 1992, Vol.175, p.217-25
(Non-Patent Document 16) Holliner P *et al.,* "Proc. Natl. Acad. Sci. USA", 1993, Vol.90, p. 6444-8
(Non-Patent Document 17) Ridgway JB *et al.,* "Protein Eng.", 1996, Vol.9, p. 617-21
(Non-Patent Document 18) Hammerling U *et al.,* "J. Exp. Med.", 1968, Vol.128, p.1461-73
(Non-Patent Document 19) Kurokawa T *et al.,* "Bio/Technology", 1989, Vol.7, p.1163
(Non-Patent Document 20) Link BK *et al.,* "Blood", 1993, Vol.81, p.3343
(Non-Patent Document 21) Nitta T *et al.,* "Lancet", 1990, Vol.335, p.368-71
(Non-Patent Document 22) deLeij L *et al.,* "Foundation Nationale de Transfusion Sanguine, Les Ulis France", 1990, p.249-53
(Non-Patent Document 23) Le Doussal JM *et al.,* "J. Nucl. Med.", 1993, Vol.34, p. 1662-71
(Non-Patent Document 24) Stickney DR *et al.,* "Cancer Res.", 1991, Vol.51, p.6650-5
(Non-Patent Document 25) Weiner LM *et al.,* "Cancer Res.", 1993, Vol.53, p.94-100
(Non-Patent Document 26) Kroesen BJ *et al.,* "Br. J. Cancer", 1994, Vol.70, p.652-61
(Non-Patent Document 27) Weiner GJ *et al.,* "J. Immunol.", 1994, Vol. 152, p.23 85
(Non-Patent Document 28) Suresh MR *et al.,* "Proc. Natl. Acad. Sci. USA", 1986, Vol.83, p.7989-93
(Non-Patent Document 29) Milstein C and Cuello AC *et al.,* "Nature", 1983, Vol.305, p.537
(Non-Patent Document 30) Xiang J *et al.,* "Mol. Immunol.", 1990, Vol.27, p.809
(Non-Patent Document 31) Bebbington CR *et al.,* "Bio/Technology", 1992, Vol.10, p. 169
(Non-Patent Document 32) Huse WD *et al.,* "Science", 1989, Vol. 246, p.1275
(Non-Patent Document 33) McCafferty J *et al.,* "Nature", 1990, Vol.348, p.552
(Non-Patent Document 34) Kang AS *et al.,* "Proc. Natl. Acad. Sci. USA", 1991, Vol.88, p. 4363

### Disclosure of the Invention

An objective of the present invention is to provide bispecific antibodies that substitute for the effect of functional proteins. More specifically, the present invention aims to provide bispecific antibodies that functionally substitute for ligands of receptors comprising heteroreceptor molecules and bispecific antibodies that functionally substitute for the cofactors which enhance enzymatic reaction.

Through dedicated research, the present inventors succeeded in separating antibodies that functionally substitute for ligands of a type I interferon receptor which comprises two types of molecules: AR1-chain and AR2-chain. In other words, for the first time, the present inventors successfully separated bispecific antibodies that can functionally substitute for ligands of heteromolecule-comprising receptors.

Through dedicated research, the present inventors succeeded in discovering bispecific antibodies that specifically bind to both F.IX/F.IXa and F.X, and substitute for the effect of cofactor F.VIIIa (i.e., a function to promote F.X activation by F.IXa). That is, the present inventors succeeded in producing bispecific antibodies that recognize both an enzyme and its substrate and functionally substitute for cofactors of the enzyme.

The above-mentioned ligand proteins of heteromolecular receptors and the above-mentioned enzyme cofactors are both functional proteins. Indeed, the present inventors have developed for the first time bispecific antibodies that functionally substitute for functional proteins.

The present invention relates to bispecific antibodies that substitute for functional proteins. More specifically, the present invention relates to bispecific antibodies that have an effect of substituting for the ligand function of heteromolecule-comprising receptors, and bispecific antibodies which functionally substitute for cofactors that enhance enzymatic reactions. More specifically, the present invention provides:
[1] A bispecific antibody that substitutes for the effect of a functional protein.
[2] A bispecific antibody that has an activity of functionally substituting for a ligand of a heteromolecule-comprising receptor.
[3] The antibody according to [2], wherein said heteromolecule-comprising receptor is a dimer.
[4] The antibody according to [2], wherein said receptor is a cytokine receptor.
[5] The antibody according to [4], wherein said cytokine receptor is an interferon receptor.
[6] The antibody according to [5], wherein said interferon receptor is a type I interferon receptor.
[7] The antibody according to [6], wherein said type I interferon receptor comprises an AR1 chain and an AR2 chain.
[8] The antibody according to [7], wherein said antibody functionally substitutes for an interferon which is a ligand of a type I interferon receptor.
[9] The antibody according to [8], wherein said antibody comprises the variable region of an anti-AR1 chain antibody and the variable region of an anti-AR2 chain antibody.
[10] The antibody according to [9], wherein said antibody comprises an anti-AR1 chain antibody variable region comprising the amino acid sequence of (a) below and an anti-AR2 chain antibody variable region comprising the amino acid sequence of any of the following (b1) to (b10):
   (a) the H chain variable region amino acid sequence described in SEQ ID NO: 1 and the L chain variable region amino acid sequence described in SEQ ID NO:2;
   (b1) the H chain variable region amino acid sequence described in SEQ ID NO: 7 and the L chain variable region amino acid sequence described in SEQ ID NO: 8;
   (b2) the H chain variable region amino acid sequence described in SEQ ID NO: 9 and the L chain variable region amino acid sequence described in SEQ ID NO: 10;
   (b3) the H chain variable region amino acid sequence described in SEQ ID NO: 19 and the L chain variable region amino acid sequence described in SEQ ID NO: 20;
   (b4) the H chain variable region amino acid sequence described in SEQ ID NO: 13 and the L chain variable region amino acid sequence described in SEQ ID NO: 14;
   (b5) the H chain variable region amino acid sequence described in SEQ ID NO: 23 and the L chain variable region amino acid sequence described in SEQ ID NO: 24;
   (b6) the H chain variable region amino acid sequence described in SEQ ID NO: 5 and the L chain variable region amino acid sequence described in SEQ ID NO: 6;
   (b7) the H chain variable region amino acid sequence described in SEQ ID NO: 17 and the L chain variable region amino acid sequence described in SEQ ID NO: 18;
   (b8) the H chain variable region amino acid sequence described in SEQ ID NO: 15 and the L chain variable region amino acid sequence described in SEQ ID NO: 16;
   (b9) the H chain variable region amino acid sequence described in SEQ ID NO: 21 and the L chain variable region amino acid sequence described in SEQ ID NO: 22;
   (b10) the H chain variable region amino acid sequence described in SEQ ID NO: 11 and the L chain variable region amino acid sequence described in SEQ ID NO: 12.
[11] The antibody according to [9], wherein said antibody comprises an anti-AR1 chain antibody variable region comprising the amino acid sequence of (a) below or an anti-AR2 chain antibody variable region comprising the amino acid sequence of any of the following (b1) to (b3):
   (a) the H chain variable region amino acid sequence described in SEQ ID NO: 3 and the L chain variable region amino acid sequence described in SEQ ID NO: 4;
   (b1) the H chain variable region amino acid sequence described in SEQ ID NO: 25 and the L chain variable region amino acid sequence described in SEQ ID NO: 26;
   (b2) the H chain variable region amino acid sequence described in SEQ ID NO: 9 and the L chain variable region amino acid sequence described in SEQ ID NO: 10;
   (b3) the H chain variable region amino acid sequence described in SEQ ID NO: 21 and the L chain variable region amino acid sequence described in SEQ ID NO: 22.
[12] A composition comprising the antibody according to any one of [2] to [11] and a pharmaceutically acceptable carrier.
[13] The composition according to [12], wherein said composition is a pharmaceutical composition used for preventing and/or treating viral disease, malignant neoplasm, or immune disease.
[14] The composition according to [13], wherein said viral disease is a disease that arises and/or progresses as a result of hepatitis C virus infection.
[15] The composition according to [14], wherein the disease that arises and/or progresses as a result of hepatitis C virus infection is acute or chronic hepatitis C, cirrhosis, or liver cancer.
[16] The composition according to [13], wherein said viral disease is a disease that arises and/or progresses as a result of hepatitis B virus infection.
[17] The composition according to [16], wherein the disease that arises and/or progresses as a result of hepatitis B virus infection is acute or chronic hepatitis B, cirrhosis, or liver cancer.
[18] The composition according to [13], wherein the malignant neoplasm is chronic myelocytic leukemia, malignant melanoma, multiple myeloma, renal cancer, gliosarcoma, medulloblastoma, astrocytoma, hairy cell leukemia, AIDS-related Kaposi's sarcoma, skin T lymphoma, or non-Hodgkin's lymphoma.
[19] The composition according to [13], wherein the immune disease is multiple sclerosis.
[20] A method for preventing and/or treating viral disease, malignant neoplasm, or immune disease, comprising the step of administering the antibody according to any one of [2] to [11], or the composition according to any one of [12] to [19].
[21] Use of the antibody according to any one of [2] to [11] for producing the composition according to any one of [12] to [19].
[22] A kit used in the method of preventing and/or treating diseases according to [20], wherein said kit comprises at least the antibody according to any one of [2] to [11], or the composition according to [12].
[23] An antibody recognizing both an enzyme and a substrate thereof, wherein said antibody is a bispecific antibody which functionally substitutes for a cofactor that enhances the enzymatic reaction.
[24] The antibody according to [23], wherein said enzyme is a proteolytic enzyme.
[25] The antibody according to [24], wherein said proteolytic enzyme, substrate, and cofactor are blood coagulation/fibrinolysis associated factors.
[26] The antibody according to [25], wherein the enzyme of a blood coagulation/fibrinolysis associated factor is blood coagulation factor IX and/or activated blood coagulation factor IX; the substrate is blood coagulation factor X; and the cofactor is blood coagulation factor VIII and/or activated blood coagulation factor VIII.
[27] The antibody according to any one of [23] to [26], wherein said antibody comprises a complementarity determining region comprising the amino acid sequence of anti-blood coagulation factor IX/IXa antibody CDR3 of the following (a1) or (a2) or a complementarity determining region functionally equivalent thereto, and a complementarity determining region comprising the amino acid sequence of anti-blood coagulation factor X antibody CDR3 described in any one of the following (b1) to (b9) or a complementarity determining region functionally equivalent thereto:
   (a1) H chain CDR3 amino acid sequence described in SEQ ID NO: 42;
   (a2) H chain CDR3 amino acid sequence described in SEQ ID NO: 46;
   (b1) H chain CDR3 amino acid sequence described in SEQ ID NO: 50;
   (b2) H chain CDR3 amino acid sequence described in SEQ ID NO: 54;
   (b3) H chain CDR3 amino acid sequence described in SEQ ID NO: 58;
   (b4) H chain CDR3 amino acid sequence described in SEQ ID NO: 62;
   (b5) H chain CDR3 amino acid sequence described in SEQ ID NO: 66;
   (b6) H chain CDR3 amino acid sequence described in SEQ ID NO: 70;
   (b7) H chain CDR3 amino acid sequence described in SEQ ID NO: 74;
   (b8) H chain CDR3 amino acid sequence described in SEQ ID NO: 78;
   (b9) H chain CDR3 amino acid sequence described in SEQ ID NO: 82.
[28] The antibody according to any one of [23] to [26], wherein said antibody comprises a complementarity determining region comprising the amino acid sequences of anti-blood coagulation factor IX/IXa antibody CDR of the following (a1) or (a2) or a complementarity determining region functionally equivalent thereto, and a complementarity determining region comprising the amino acid sequence of anti-blood coagulation factor X antibody CDR described in any one of the following (b1) to (b9) or a complementarity determining region functionally equivalent thereto:
   (a1) H chain CDR 1,2, and 3 amino acid sequences described in SEQ ID NOs: 40,41, and 42, respectively;
   (a2) H chain CDR 1, 2, and 3 amino acid sequences described in SEQ ID NOs: 44, 45, and 46, respectively;
   (b1) H chain CDR 1, 2, and 3 amino acid sequences described in SEQ ID NOs: 48, 49, and 50, respectively;
   (b2) H chain CDR 1, 2, and 3 amino acid sequences described in SEQ ID NOs: 52, 53, and 54, respectively;
   (b3) H chain CDR 1, 2, and 3 amino acid sequences described in SEQ ID NOs: 56, 57, and 58, respectively;
   (b4) H chain CDR 1, 2, and 3 amino acid sequences described in SEQ ID NOs: 60, 61, and 62, respectively;
   (b5) H chain CDR 1, 2, and 3 amino acid sequences described in SEQ ID NOs: 64, 65, and 66, respectively;
   (b6) H chain CDR 1, 2, and 3 amino acid sequences described in SEQ ID NOs: 68, 69, and 70, respectively;
   (b7) H chain CDR 1, 2, and 3 amino acid sequences described in SEQ ID NOs: 72, 73, and 74, respectively;
   (b8) H chain CDR 1, 2, and 3 amino acid sequences described in SEQ ID NOs: 76, 77, and 78, respectively;
   (b9) H chain CDR 1, 2, and 3 amino acid sequences described in SEQ ID NOs: 80, 81, and 82; respectively.
[29] A composition comprising the antibody according to any one of [23] to [28] and a pharmaceutically acceptable carrier.
[30] The composition according to [29], wherein said composition is a pharmaceutical composition used for preventing and/or treating bleeding, disorder accompanied by bleeding, or disorder caused by bleeding.
[31] The composition according to [30], wherein the bleeding, disorder accompanied by bleeding, or disorder caused by bleeding is a disorder that arises and/or progresses as a result of an activity decrease or deficiency of blood coagulation factor VIII and/or activated blood coagulation factor VIII.
[32] The composition according to [31], wherein the disorder that arises and/or progresses as a result of an activity decrease or deficiency of blood coagulation factor VIII and/or activated blood coagulation factor VIII is hemophilia A.
[33] The composition according to [31], wherein the disorder that arises and/or progresses as a result of an activity decrease or deficiency of blood coagulation factor VIII and/or activated blood coagulation factor VIII is a disorder in which an inhibitor against blood coagulation factor VIII and/or activated blood coagulation factor VIII is generated.
[34] The composition according to [31], wherein the disorder that arises and/or progresses as a result of an activity decrease or deficiency of blood coagulation factor VIII and/or activated blood coagulation factor VIII is acquired hemophilia.
[35] The composition according to [31], wherein the disorder that arises and/or progresses as a result of an activity decrease of blood coagulation factor VIII and/or activated blood coagulation factor VIII is von Willerbrand's disease.
[36] A method for preventing and/or treating bleeding, disorder accompanied by bleeding, or disorder caused by bleeding, wherein said method comprises the step of administering the antibody according to any one of [23] to [28], or the composition according to any one of [29] to [35].
[37] Use of the antibody according to any one of [23] to [28] for preparing the composition according to any one of [29] to [35].
[38] A kit used in the method of preventing and/or treating disorders according to [36], wherein said kit comprises at least the antibody according to any one of [23] to [28] or the composition according to [29].

A bispecific antibody according to the present invention is a molecule comprising two types of antibodies or antibody fragments having specificities for different antigens. The bispecific antibody is not particularly limited, but preferably monoclonal.

The bispecific antibodies of the present invention are preferably recombinant antibodies generated using gene recombination techniques (see e.g. Borrebaeck CAK and Larrick JW, THERAPEUTIC MONOCLONAL ANTIBODIES, Published in the United Kingdom by MACMILLAN PUBLISHERS LTD, 1990). A recombinant antibody can be obtained by cloning an antibody-encoding DNA from antibody-producing cells, such as hybridomas or sensitized lymphocytes, incorporating the DNA into an appropriate vector, and introducing the vector into a host for antibody production.

Further, antibodies of the present invention may be antibody fragments or modified antibodies. Antibody fragments may include diabody (Db), linear antibody, single-strand antibody (hereinafter also referred to as scFv) molecules, etc. Herein, "Fv" fragment represents the smallest antibody fragment, comprising a complete antigen-recognizing site and binding site. An "Fv" fragment is a dimer (V_{H}-V_{L} dimer) in which one heavy (H) chain variable region (V_{H}) and one light (L) chain variable region (V_{L}) are strongly connected by a non-covalent bond. Three complementarity determining region (CDRs) of each variable region interact to form an antigen-binding site on the surface of a V_{H}-V_{L} dimer. Six CDRs confer an antigen-binding site on an antibody. However, even one variable region (or half of an Fv, which contains only three antigen specific CDRs) is capable of recognizing an antigen and binding thereto, although its affinity is lower than that of the entire binding site.

In addition, Fab fragment (also referred to as (F(ab)) further contains an L chain constant region and an H chain constant region (CH1). A Fab' fragment differs from a Fab fragment in that the former contains several additional residues derived from the carboxyl terminal of an H chain CH1 region, which comprises one or more cysteines from the hinge region of an antibody. Fab'-SH refers to Fab' having a free thiol group in one or more cysteine residues of the constant region. F(ab') fragments are generated by cleaving the disulfide bond in the cysteines of the hinge portion of an F(ab')₂ pepsin digest. Other chemically bound antibody fragments are also known to those skilled in the art.

Diabody refers to a bivalent antibody fragment constructed by gene fusion (Holliger P *et al.,* Proc. Natl. Acad. Sci. USA 90: 6444-6448 (1993); EP 404,097; WO 93/11161; etc.). Diabody is a dimer comprising two peptide chains; in each polypeptide chain, an L chain variable region (V_{L}) is connected to an H chain variable region (V_{H}) on the same chain via a linker that is too short to allow paring between the two regions (for example, about 5 residues). V_{L} and V_{H} encoded on the same polypeptide chain form a dimer because they cannot form a single-stranded variable region fragment due to the short linker between them. Thus, a diabody ends up with two antigen binding sites.

A single-strand antibody or scFv fragment contains the V_{H} and V_{L} regions of an antibody, and these regions exist in a single polypeptide chain. In general, an Fv polypeptide further contains a polypeptide linker between V_{H} and V_{L} regions, such that scFv is able to form a structure that is necessary for antigen binding (see Pluckthun "The Pharmacology of Monoclonal Antibodies" Vol. 113 (Rosenburg and Moore ed (Springer Verlag, New York) pp. 269-315, 1994 for general remarks on scFv). The linkers of the present invention are not particularly limited, as long as they do not inhibit expression of the antibody variable regions connected to both ends of a linker.

An IgG type bispecific antibody can be secreted by a hybrid hybridoma (quadroma) formed by fusing two types of hybridomas that produce IgG antibodies (Milstein C *et al.,* Nature 1983, 305: 537-540). It can also be secreted by introducing into cells genes of the L chains and H chains that constitute the two IgGs of interest (a total of four types of genes) for co-expression. In this case, by appropriately substituting amino acid(s) in the CH3 region of an H chain, it is possible to preferentially secrete IgGs that have a heterologous combination of H chains (Ridgway, JB *et al.* Protein Engineering 1996, 9: 617-621, Merchant, AM *et al.* Nature Biotechnology 1998, 16: 677-681).

A bispecific antibody can also be prepared by chemically cross-linking Fab's. A bispecific F(ab')₂ can be produced, for example, by maleimidating a Fab' prepared from one antibody with o-PDM (ortho-phenylenedi-maleimide) and reacting the product with a Fab' prepared from another antibody, so as to cross-link Fab's derived from different antibodies (Keler T *et al.* Cancer Research 1997, 57: 4008-4014). Further, a method for chemically connecting antibody fragments such as a Fab'-thionitrobenzoic acid (TNB) derivative and Fab'-thiol (SH) is also known (Brennan M *et al.* Science 1985, 229: 81-83).

Instead of cross linkage, a leucine zipper derived from Fos and Jun or the like can be used. Although Fos and Jun also form a homodimer, their preferential heterodimer formation is utilized. A Fab' added with a Fos leucine zipper and a second Fab' added with a Jun leucine zipper is expressed for preparation. By mixing and reacting monomeric Fab'-Fos and Fab'-Jun, which have been reduced under mild conditions, a bispecific F(ab')₂ can be formed (Kostelny SA *et al.* J. of Immunology, 1992, 148: 1547-53). This method is not limited to Fab' and can also be applied to scFv, Fv, etc.

A bispecific antibody can also be prepared in a form of diabody. A bispecific diabody is a heterodimer comprising two crossover scFv fragments. That is, a bispecific diabody can be prepared by constructing a heterodimer using V_{H}(A)-V_{L}(B) and V_{H}(B)-V_{L}(A), which have been formed by connecting V_{H} and V_{L} derived from two types of antibodies: A and B, with a relatively short linker of about 5 amino acid residues (Holliger P *et al.* Proc. of the National Academy of Sciences of the USA 1993, 90: 6444-6448).

In this case, construction of a bispecific diabody of interest can be promoted by performing appropriate amino acid substitutions (knobs-into-holes: Zhu Z *et al.* Protein Science. 1997, 6: 781-788) so as to link two types of scFv's with a flexible and relatively long linker of about 15 amino acid residues (a single-chain diabody: Kipriyanov SM *et al.* J. of Molecular Biology. 1999, 293: 41-56).

sc(Fv)₂ which can be prepared by linking two types of scFv's with a flexible and relatively long linker of about 15 amino acid residues can also become a bispecific antibody (Mallender WD *et al.* J. of Biological Chemistry, 1994, 269: 199-206).

A modified antibody may be, for example, an antibody that binds to various molecules such as polyethylene glycol (PEG). In the modified antibodies of the present invention, substances to be bound are not limited. Such modified antibodies can be obtained by chemically modifying the antibodies obtained. These methods have already been established in this field.

The antibodies of the present invention include human antibody, mouse antibody, rat antibody and such, without any limitation on their origins, and may be genetically modified antibodies such as chimera antibody and humanized antibody.

Methods for obtaining human antibodies are known, and a human antibody of interest can be obtained, for example, by immunizing a transgenic animal having all repertoires of human antibody genes with an antigen of interest (see WO 93/12227, WO 92/03918, WO 94/02602, WO 94/25585, WO 96/34096, WO 96/33735).

Genetically modified antibodies can be produced by known methods. Specifically, for example, a chimera antibody comprises variable regions from the H and L chains of an antibody from immunized animals, and constant regions from the H and L chains of a human antibody. A chimera antibody can be obtained by linking a DNA encoding the variable region of an antibody derived from immunized animals with a DNA encoding the constant region of a human antibody, inserting the resulting DNA into an expression vector, and introducing the recombinant vector into a host for production.

A humanized antibody is a modified antibody also referred to as reshaped human antibody. A humanized antibody is constructed by grafting the complementarity determining region (CDR) of an antibody derived from immunized animals into the CDR of a human antibody. General genetic engineering technologies are also known.

Specifically, a DNA sequence designed to link the CDR of a mouse antibody to the framework region (FR) of a human antibody is synthesized by PCR, using several oligonucleotides that have been prepared to contain overlapping portions at their terminal regions. After linking the obtained DNA to a DNA encoding the constant region of a human antibody, the resulting DNA is incorporated into an expression vector and introduced into a host to produce a humanized antibody (see EP 239400 and WO 96/02576). As a human antibody FR to be linked via CDR, one that is capable of forming an antigen-binding site with a good complementarity determining region is selected. Amino acids of the framework region in an antibody variable region may be substituted as necessary, so that the complementarity determining region of a reshaped human antibody forms an appropriate antigen-binding site (Sato K *et al,* Cancer Research 1993, 53: 851-856). The framework region may be substituted with framework regions derived from various human antibodies (see WO 99/51743).

The present invention provides bispecific antibodies that functionally substitute for functional proteins, more preferably, bispecific antibodies that functionally substitute for functional proteins. A preferred embodiment of the antibodies of the present invention is an antibody that has an activity to functionally substitute for heteromolecule-comprising receptors.

In the present invention, heteromolecule-comprising receptors refer to receptors (multimer) composed of two or more different proteins (receptor molecules). Multimers are not limited by the number of proteins (receptor molecules) and include dimers, trimers, tetramers, etc., but are preferably dimers. For example, in the case of a dimer receptor, a heteroreceptor indicates that the two constitutional proteins (receptor molecule) are not identical.

Antibodies having an activity to functionally substitute for ligands refer to antibodies that have an agonistic action against certain receptors. In general, when a ligand (i.e., an agonist) binds to a receptor, the tertiary structure of the receptor protein changes and the receptor is activated (in the case of a membrane protein receptor, cell proliferation signals and such are usually emitted). When the receptor type is one that forms a dimer, antibodies that functionally substitute for the ligand can work similarly to a ligand by dimerizing the receptor at an appropriate distance and angle. In other words, anti-receptor antibodies can mimic ligand-induced dimerization of receptors, and become antibodies that functionally substitute for ligands.

In a preferred embodiment of the present invention, the receptor of the present invention is a cytokine heteroreceptor.

The term "cytokine" is normally used as a collective term to refer to bioactive proteins that regulate the proliferation and differentiation of various types of hemocytes. It is also used to refer to growth factors and growth inhibitory factors of cells including non-immune cells. Therefore, the term "cytokine" collectively refers to cell-released proteinaceous factors that mediate cell-cell interactions such as regulation of immunoreaction and inflammatory response, antiviral actions, antitumor actions, and regulation of cell proliferation and/or differentiation.

Specific examples of cytokines that act on heteroreceptors of the present invention include IL-2, 3, 4, 5, 6, 7, 9, 10, 11, 12, 13, and 15, colony-stimulating factors (GM-CSF, etc.), interferons (IFN-α, IFN-β, IFN-γ, etc.), CNTF, LIF, Oncostatin M, CT-1, and such, but are preferably interferons, especially type I interferons.

Interferons include IFN-α, IFN-β, IFN-γ, IFN-τ, etc. IFN-α and IFN-β are highly homologous and thus, these two IFNs can react via a same receptor. Furthermore, IFN-α, IFN-β, and IFN-τ are classified as type I interferon.

Examples of type I interferon receptors include receptors having an AR1 chain (GenBank ACCESSION No: J03171, literature: Uze G *et al.* Cell 1990, 60: 225-34) and an AR2 chain (GenBank ACCESSION No: U29584, literature: Domanski P *et al.* J of Biological Chemistry 1995, 270: 21606-11, LutfaUa G *et al.* EMBO J 1995, 14: 5100-8).

The method for obtaining bispecific antibodies that functionally substitute for ligands of the present invention are not particularly limited, and may be obtained by any method. For example, to obtain a bispecific antibody that functionally substitutes for a ligand of a heteroreceptor comprising two types of receptor molecules (A chain and B chain), anti-A chain antibody and anti-B chain antibody are first obtained. Subsequently, a bispecific antibody comprising the H chain and L chain of the anti-A chain antibody, and the H chain and L chain of anti-B chain antibody is produced. Preferably, multiple types of anti-A chain antibodies and anti-B chain antibodies are obtained to produce as many combinations of bispecific antibodies as possible. Bispecific antibodies are produced, and then those that have an activity to functionally substitute for ligands are selected. Bispecific antibodies may be produced by known methods such as fusion of antibody-producing hybridomas, or introduction of antibody expressing vectors into cells.

Antibodies against receptors may be obtained by methods known to a person skilled in the art. For example, antibodies may be prepared by immunizing immune animals with antigens. Antigens that are used for animal immunization include complete antigens having immunogenicity, and incomplete antigens lacking immunogenicity (including haptens). In the present invention, a receptor whose ligand is functionally substituted by an antibody of the present invention presumed to act as the ligand is used as an antigen (immunogen) mentioned above. The above-mentioned receptor of the present invention is not particularly limited, but is preferably a heterodimer. For example, mice, hamsters, or rhesus monkeys may be used as an immune animal. These animals may be immunized with antigens by a person skilled in the art, using well known methods. In the present invention, variable regions of the L chain and H chain are preferably recovered from immunized animals, or cells of the animals. This process may be carried out by methods generally known to a person skilled in the art. Animals immunized with an antigen express antibodies against the antigen, especially in their spleen cells. For example, mRNAs may be prepared from spleen cells of the immunized animals, and the L chain and H chain variable regions may be recovered by RT-PCR using primers that correspond to the variable region of the animal.

Specifically, the A chain and B chain are each used to immunize an animal. Receptors used as the immunogen may be a whole protein constituting a receptor, or a partial peptide of the protein. Further, the immunogen used for animal immunization may be made into a soluble immunogen by binding an antigenic molecule, or fragments thereof, with other molecules. When transmembrane molecules, such as receptors, are used as an antigen, it is preferable to use their fragments (for example, extracellular region of a receptor). Cells expressing a transmembrane molecule on their cell surface may also be used as an antigen. Such cells may be naturally occurring cells (tumor cell lines etc.) or cells constituted by genetic recombination techniques to express transmembrane molecules. mRNAs are extracted from spleen cells of an immunized animal, and cDNAs of the L chain and H chain variable regions are recovered by RT-PCR using primers corresponding to regions in the vicinity of the variable regions. Primers corresponding to CDR, primers corresponding to framework regions which are less diversified than CDR, or primers corresponding to signal sequences and CH1 or L chain constant region (C_{L}) may also be used. Lymphocytes may be immunized *in vitro* and used to construct scFv- or Fab-presenting libraries. Clones of antigen-bound antibodies are concentrated by panning and cloned, and antibody expression vectors are produced using their variable regions. Anti-A chain antibody expression vector and anti-B chain antibody expression vector are introduced into a same cell, and by expressing the antibodies, a bispecific antibody is obtained. In this case, screening may be performed using similar mRNA libraries derived from human peripheral blood mononuclear cells, spleen, tonsil and such, or those of unimmunized animals.

Antibodies that have an activity to functionally substitute for ligands may be selected, for example, by the following methods.
(1) Add an antibody to a culture of cells that proliferate in a ligand-dependent manner, check whether or not the cells proliferate as in the case of ligand addition, and use it as an indicator. If the cells proliferate, the subject multispecific antibody is judged to have an effect of functionally substituting for the ligand.
(2) Add an antibody to the culture of a cell line that reflects the original activity of a ligand (but not necessarily proliferation), check whether or not the cells react to the added antibody the same way as to the ligand and use it as an indicator. If the cells react the same way as how they react to the ligand, the antibody is judged to have an effect of functionally substituting for the ligand.

The above cells normally express on their cell surface heteroreceptors against which antibodies can act as agonists, and the receptors bind to ligands to emit signals. Cells used in the above method are preferably cells that can proliferate dependently on receptor ligands (ligand-dependent proliferating cells). Normally, the above receptors are preferably those that emit cell proliferation signals by binding to a ligand. However, if the above receptor is one that does not emit cell proliferation signals, the receptor can be fused with a type of receptor that emits cell proliferation signals to form a so-called chimeric receptor, for use in the above methods. The chimeric receptor emits cell proliferation signals by binding with a ligand. Receptors that are suitable for the construction of chimeric receptors by fusing with a receptor are not particularly limited as long as they are a type of receptor that emits cell proliferation signals. They are normally membrane proteins, and preferably, receptors comprising a receptor fragment with a ligand-binding function (extracellular region), and a receptor fragment with a signal transduction function (intracellular region). Receptors used for the intracellular region are specifically GH receptor, G-CSF receptor, MPL, EPO receptor, c-Kit, Flt-3 IL-2 receptor, IL-3 receptor, IL-5 receptor, GM-CSF receptor and such. Specifically, suitable examples of the above ligand-dependent proliferating cells of the present invention include, ligand-dependent proliferating cell Ba/F3, which expresses chimeric receptors in which the extracellular region is a ligand receptor fragment and the intracellular region is a G-CSF receptor fragment. Examples of cells that can be used in the above methods include, for example, NFS60, FDC-P1, FDC-P2, CTLL-2, DA-1, and KT-3.

The antibodies thus obtained may be purified to homogeneity. Separation and purification of antibodies may be performed by separation and purification methods used for general proteins. Without being limited thereto, antibodies can be separated and purified by, for example, arbitrarily selecting and combining chromatography columns such as affinity chromatography, filters, ultrafiltration, salt precipitation, dialysis, SDS polyacrylamide gel electrophoresis, and isoelectric focusing (Antibodies: A Laboratory Manual. Ed Harlow and David Lane, Cold Spring Harbor Laboratory, 1988). Columns used for affinity chromatography include protein A column, protein G column and such.

When the antibody of the present invention is, for example, an antibody that has an effect of functionally substituting for a ligand of a type I interferon receptor comprising an AR1 chain and an AR2 chain, the antibody preferably has a structure comprising the variable region of an anti-AR1chain antibody and the variable region of an anti-AR2 chain antibody. An antibody that functionally substitutes for interferon was produced by the following method. IL-3 dependent mouse proB cell line Ba/F3, which expresses a chimeric receptor comprising the intracellular region of G-CSF receptor and the extracellular region of either of the receptor molecules (AR1 chain and AR2 chain) of the type I interferon receptor, was established. BALB/c was intraperitoneally immunized with either of the cells.

PolyA(+)RNA was extracted from the spleen of an immunized mouse with an elevated antibody titer, scFv was synthesized by RT-PCR, and an scFv presenting phage library was constructed. After mixing a phage library derived from the spleen of an AR1 chain-expressing Ba/F3 immunized mouse and biotinylated soluble AR1 chain, bound phages were concentrated by a panning method, which captures the phages by streptavidin magnetic beads. Phages presenting the anti-AR1 chain antibody were selected by ELISA using soluble AR1 chain. Similarly, anti-AR2 chain antibody phages were selected using soluble AR2 chain and library phages derived from the spleen of an AR2 chain-expressing Ba/F3 immunized mouse. Antibodies comprising a different amino acid sequence for the H chain CDR3, which is thought to be most involved in antibody specificity, were selected.

An scFv-CH1-Fc expression vector was produced by inserting scFv between a signal sequence for animal cells and CH1-hinge-CH2-CH3. Anti-AR1 chain antibodies and anti-AR2 chain antibodies were introduced into cells in various combinations for the expression of bispecific antibodies.

BaF3-ARG was established by introducing into Ba/F3, expression vectors of chimeric molecules comprising the extracellular region of AR1 chain or AR2 chain and the intracellular region of G-CSF receptor. These cells proliferated IFN-α dependently. Bispecific antibodies comprising an antibody combination that could support BaF3-ARG proliferation were selected.

Daudi cells are a human B-cell line that is highly sensitive to cell growth inhibition activity by IFN-α. The earlier selected bispecific antibodies were added to Daudi cells and confirmed to inhibit proliferation like IFN-α. The antibodies are not particularly limited and include, for example, antibodies comprising either of the following anti-AR1 chain antibody variable regions, or one of the following anti-AR2 chain antibody variable regions.
- Variable regions of anti-AR1 chain antibody: AR1-41, AR1-24
- Variable regions of anti-AR2 chain antibody: AR2-37, AR2-11, AR2-13, AR2-45, AR2-22, AR2-43, AR2-40, AR2-14, AR2-44, AR2-33, and AR2-31

Amino acid sequences of the V_{H} and V_{L} for each of the above-mentioned variable regions are shown in SEQ ID NOs: 1 to 26 (correlation between the variable regions V_{H} and V_{L} and the SEQ ID NOs: is shown in Table 1 below).

**Table 1**

| Variable region | SEQ ID NO: | |
|---|---|---|
| | V_{H} | V_{L} |
| AR1-41 | 1 | 2 |
| AR1-24 | 3 | 4 |
| AR2-37 | 5 | 6 |
| AR2-11 | 7 | 8 |
| AR2-13 | 9 | 10 |
| AR2-45 | 11 | 12 |
| AR2-22 | 13 | 14 |
| AR2-43 | 15 | 16 |
| AR2-40 | 17 | 18 |
| AR2-14 | 19 | 20 |
| AR2-44 | 21 | 22 |
| AR2-33 | 23 | 24 |
| AR2-31 | 25 | 26 |

When the anti-AR1 chain antibody is AR1-24, its partner anti-AR2 chain antibody is preferably AR2-13, AR2-31, or AR2-44, and when the anti-AR1 chain antibody is AR1-41, its partner anti-AR2 chain antibody is preferably AR2-11, AR2-13, AR2-14, AR2-22, AR2-33, AR2-37, AR2-40, AR2-43, AR2-44, or AR2-45. AR2-13 and AR2-44 can become a partner for both AR1-41 and AR1-24 antibodies. The present invention also includes antibodies that form pairs as shown above.

A preferred embodiment of a bispecific antibody that functionally substitutes for a functional protein of the present invention is a bispecific antibody that functionally substitutes for a cofactor that recognizes both an enzyme and its substrate.

Cofactors of the present invention are not particularly limited, as long as they are capable of acting on an enzyme to enhance the enzymatic reaction. A cofactor of the present invention is, for example, a cofactor of a proteolytic enzyme. Specific examples of a cofactor of a proteolytic enzyme are cofactors for blood coagulation and fibrinolysis associated factors (F.VIII/F.VIIIa, PZ, TM, TM/PS system), cofactors for complement reactions (C4b, MCP, CR1, H factor), and such.

The following combinations can be listed as specific examples of enzyme and enzyme substrate, as well as enzyme cofactors.

### (a) Cofactor for blood coagulation and fibrinolysis associated factor (Example 1)

- Enzyme:: F.IXa
- Substrate:: F.X
- Cofactor:: F.VIII/F.VIIIa

Cofactor F.VIIIa binds to both F.IXa and F.X and enhances F.X activation by F.IXa. Among bispecific antibodies that recognize both the above-described enzyme F.IXa and substrate F.X, some have an enhancing effect on F.X activation. Some of these antibodies are thought to have an effect of substituting for the function of cofactor F.VIII/F.VIIIa.

### (b) Cofactor for blood coagulation and fibrinolysis associated factor (Example 2)

- Enzyme:: ZPI
- Substrate:: F.X/F.Xa
- Cofactor:: PZ

Cofactor PZ binds to ZPI of the serpin family and F.Xa to enhance the F.Xa-inhibiting activity of ZPI. Specifically, some bispecific antibodies that recognize both ZPI and F.X/F.Xa are thought to have an effect of substituting for the PZ function.

### (c) Cofactor for blood coagulation and fibrinolysis associated factor (Example 3)

- Enzyme:: thrombin
- Substrate:: TAFI
- Cofactor:: TM

Cofactor TM enhances TAFI activation by thrombin. Specifically, some bispecific antibodies that recognize both thrombin and TAFI are thought to have an effect of substituting for the TM function.

### (d) Cofactors for blood coagulation and fibrinolysis associated factor (Example 4)

- Enzyme:: thrombin
- Substrate:: PC
- Cofactors:: TM/PS

The TM/PS system enhances PC activation by thrombin. Specifically, some bispecific antibodies that recognize both thrombin and PC are thought to functionally substitute for the TM/PS system.

### (e) Cofactor for complement reactions (Example 1)

- Enzyme:: C1s
- Substrate:: C2
- Cofactor:: C4b

C4b has C1s' promoting effect on C2 decomposition. That is, among the bispecific antibodies that recognize both C1s and C3, some are thought to functionally substitute for C4b.

### (f) Cofactors for complement reactions (Example 2)

- Enzyme:: Complement Regulatory Factor I
- Substrate:: C3b
- Cofactors:: Complement Regulatory Factor H,
Membrane Cofactor Protein (MCP), and
Complement Receptor 1 (CR1)

Complement Regulatory Factors H, MCP, and CR1 have the promoting effect of Complement Regulatory Factor 1 on C3b degradation. Specifically, among bispecific antibodies that recognize both Complement Regulatory Factor 1 and C3b, some are thought to functionally substitute for Complement Regulatory Factors H, MCP, and CR1.

Among the above-described cofactors, F.VIII/F.VIIIa is particularly preferable. Although F.VIII/F.VIIIa undergoes limited proteolysis by proteolytic enzymes such as thrombin, as long as it has F.VIII/F.VIIIa activity, its form does not matter. Further, F.VIII/F.VIIIa variants and F.VIII/F.VIIIa that have been artificially modified by gene recombination techniques are also included in F.VIII/F.VIIIa, as long as they retain F.VIII/F.VIIIa cofactor activity.

Methods for obtaining bispecific antibodies which functionally substitute for cofactors of the present invention are not particularly limited, and may be obtained by any methods. For example, when obtaining a bispecific antibody that functionally substitutes for enzyme A and substrate B, enzyme A and substrate B are each immunized to an animal to obtain anti-enzyme A antibody and anti-substrate B antibody. Subsequently, a bispecific antibody comprising the anti-enzyme A antibody H and L chains and the anti-substrate B antibody H and L chains is produced. Herein, it is desirable to obtain several types of each of the anti-enzyme A antibody and the anti-substrate B antibody, such that these antibodies can be preferably used to produce as many combinations of bispecific antibodies as possible. After bispecific antibodies are produced, antibodies with an activity that substitutes for cofactor function are selected.

Antibodies against an enzyme or a substrate can be obtained by methods known to those skilled in the art. For example, antibodies can be prepared by immunizing animals with antigens. Antigens for immunizing animals are, for example, complete antigens having immunogenicity and incomplete antigens (including hapten) without immunogenicity. In the present invention, an enzyme whose cofactor can be functionally substituted by an antibody of the present invention which acts as the cofactor, or a substrate of the enzyme, is used as the above-described antigen (immunogen). As animals to be immunized, for example, mouse, hamster, or rhesus monkey can be used. Immunization of these animals with antigens can be performed by methods known to those skilled in the art. In the present invention, antibody L chain and H chain variable regions are preferably collected from immunized animals or cells thereof. This procedure can be performed by one skilled in the art by using generally known methods. Antigen-immunized animals express antibodies against the antigen, especially in the spleen cells. Therefore, for example, mRNA can be prepared from spleen cells of an immunized animal, and variable regions of the L chain and H chain can be recovered by RT-PCR using primers to the animal's variable regions.

Specifically, animals are immunized with an enzyme or a substrate. The enzyme and substrate used as immunogens may be whole proteins or partial peptides thereof. Further, depending on the circumstances, a candidate antigen bound to another molecule to form a soluble antigen, or fragments of which, may be used as an immunogen for immunizing animals.

mRNA is extracted from the spleen cells of immunized animals, and cDNAs of the L chain and H chain variable regions are recovered by RT-PCR, using primers to the vicinity of the variable regions. Primers to CDR, primers to framework regions which are less diversified than CDR, or primers to signal sequences and CH1 or L chain constant region (C_{L}) may also be used. Further, lymphocytes can also be immunized *in vitro,* and used to construct scFv or Fab presenting libraries. Antigen-binding antibody clones are concentrated and cloned by panning, and their variable regions are used to produce antibody expression vectors. By introducing an anti-enzyme antibody expression vector and an anti-substrate antibody expression vector into a same cell and expressing the antibodies, a bispecific antibody can be obtained. In this case, screening can also be performed using similar libraries constructed from mRNAs derived from the peripheral blood monocytes, spleen, tonsil and such of human and non-immunized animals as materials.

Antibodies that have a cofactor function-substituting activity can be selected, for example, by the methods described below.
(1) In a reaction system comprising the enzyme and the substrate, the selection is performed using elevation of enzyme activity (substrate degradation ability) as an index, wherein the elevation of enzyme activity is a result of antibody addition.
(2) In a system for measuring or simulating the biological functions which the enzyme, substrate, and cofactor are involved in (for example, a system for measuring plasma coagulation), the selection is performed using activity of functional recovery as an index, wherein the activity of functional recovery is a result of antibody addition in the absence of the cofactor.

The antibody thus obtained can be purified to homogeneity. Separation and purification of the antibody may be performed by separation and purification methods used for general proteins. For example, antibodies can be separated and purified by appropriately selecting and combining chromatography columns such as affinity chromatography, filter, ultrafiltration, salting out, dialysis, SDS polyacrylamide gel electrophoresis, isoelectric electrophoresis and so on (Antibodies: A Laboratory Manual. Ed Harlow and David Lane, Cold Spring Harbor Laboratory, 1988), but the methods are not limited thereto. A column used in affinity chromatography is, for example, protein A column, protein G column and such.

For example, when F.VIII/F.VIIIa is the substitute cofactor, that is, when the enzyme and substrate combination is plasma coagulation and fibrinolysis associated factors F.IXa and F.X, the bispecific antibody of the present invention preferably has a structure comprising the variable region of an anti-F.IXa antibody and the variable region of an anti-F.X antibody.

Bispecific antibodies of the present invention which functionally substitute for F.VIII/F.VIIIa were generated by the following method. Mice were subcutaneously immunized with commercial F.IXa or F.X. Spleen cells were isolated from spleens of the immunized mice with an elevated antibody titer, and fused with mouse myeloma cells to form hybridomas. Hybridomas that bind to antigen F.IXa or F.X were selected, and the L chain and H chain variable regions were recovered by RT-PCR, using primers to the variable regions. The L chain variable region was incorporated into a C_{L}-containing L chain expression vector, and the H chain variable region was inserted into an H chain expression vector containing an H chain constant region.

The anti-F.IXa antibody (H chain, L chain) expression vectors and anti-F.X antibody (H chain, L chain) expression vectors were introduced into same cells for antibody expression and bispecific antibodies were obtained.

Bispecific antibodies thus obtained were assessed for their effects to functionally substitute for F.VIII/F.VIIIa (cofactors for F.X activation by F.IXa) in an assay system comprising F.XIa (F.IX activating enzyme), F.IX (F.X activating enzyme), F.X, a synthetic substrate (S-2222) for F.Xa, and phospholipid. Given this result, bispecific antibodies having the activity to substitute for F.VIII/F.VIIIa were selected.

The bispecific antibodies selected above were measured for their ability to restore coagulation in a coagulation assay system (APTT) that uses human F.VIII-deficient plasma. The results confirmed that bispecific antibodies which have the ability to restore coagulation in human F.VIII-deficient plasma were obtained.

The H chain CDR3s of the present invention's antibodies are not particularly limited, but specifically have a complementarity determining region comprising either the amino acid sequence of the XB12 H chain CDR3 sequence (SEQ ID NO: 42) or the XT04 H chain CDR3 sequence (SEQ ID NO: 46) described below in the examples, or those functionally equivalent thereto, and the complementarity determining region comprising an amino acid sequence described in any one of the H chain CDR3 sequences (SEQ ID NOs: 50, 54, 58, 62, 66, 70, 74, 78, and 82) in SB04, SB05, SB06, SB07, SB21, SB30, SB34, SB38, and SB42, respectively, or those functionally equivalent thereto.

Further, a specific example of the above-described antibodies is preferably combined from an antibody having a complementarity determining region comprising either an H chain CDR sequence of XB12 (SEQ ID NOs: 40-42) or an H chain CDR sequence of XT04 (SEQ ID NOs: 44-46), or a complementarity determining region functionally equivalent thereto, and an antibody having a complementarity determining region comprising any one of the H chain CDR sequences (SEQ ID NOs: 48-50, 52-54, 56-58, 60-62, 64-66, 68-70, 72-74, 76-78, or 80-82) in SB04, SB05, SB06, SB07, SB21, SB30, SB34, SB38, and SB42, respectively, or a complementarity determining region functionally equivalent thereto.

The amino acid sequences of the H chain variable regions of XB 12, XT04, SB04, SB05, SB06, SB07, SB21, SB30, SB34, SB38, and SB42 disclosed in the present invention are shown as SEQ ID NOs: 39, 43, 47, 51, 55, 59, 63, 67, 71, 75, and 79.

When preparing a full-length antibody using the variable regions disclosed in the present invention, the constant regions are not particularly limited, and those known to one skilled in the art, for example, constant regions described in "Sequences of proteins of immunological interest, (1991), U.S. Department of Health and Human Services. Public Health Service National Institutes of Health" and "An efficient route to human bispecific IgG, (1998). Nature Biotechnology vol. 16, 677-681", and such can be used.

In one embodiment of the antibodies of the present invention, the antibody of the present invention is expected to, through its ligand function-substituting effect, become an effective drug against diseases caused by a decrease in the activity (function) of the receptor on which the antibody acts.

When the ligand for which the antibody of the present invention functionally substitutes is IFN-α/β, the above diseases include, for example, viral diseases, malignant neoplasms, and immune diseases.

Viral diseases include, for example, diseases that arise and/or progress via hepatitis C virus, and more specifically, acute hepatitis C, chronic hepatitis C, cirrhosis, liver cancer and such.

Chronic hepatitis C is a chronic inflammatory disease caused by host immune response against hepatitis C virus-infected cells. As the symptoms progress, liver function gradually decreases and through cirrhosis, leads to liver cancer at the end. In order to eliminate hepatitis C virus from chronic hepatitis C patients, interferon-α/β therapy is carried out. However, due to their short half life in blood, daily administration is required and thus places a considerably heavy burden on the patient. Therefore, drugs which have the interferon-α/β effect and an outstanding sustainability are in demand.

Other examples of viral diseases include diseases that arise and/or progress via hepatitis B virus, and more specifically, acute hepatitis B, chronic hepatitis B, cirrhosis, liver cancer and such.

Examples of malignant neoplasms include chronic myelocytic leukemia, malignant melanoma, multiple myeloma, renal cancer, gliosarcoma, medulloblastoma, astrocytoma, hairy cell leukemia, AIDS related Kaposi's sarcoma, skin T lymphoma, and non Hodgkin's lymphoma.

An example of an immune disease is multiple sclerosis.

In other embodiments, the antibodies of the present invention have an effect to functionally substitute for cofactors, and are thus expected to become effective drugs for diseases caused by decrease in the activity (function) of these cofactors. In cases where the cofactor functionally substituted by an antibody of the present invention is a blood coagulation and fibrinolysis-associated factor, the above-described diseases are, for example, bleeding, diseases accompanied by bleeding, diseases caused by bleeding, and such. In particular, functional reduction and deficiency in F.VIII/F.VIIIa, F.IX/F.IXa, and F.XI/F.XIa have been known to cause abnormal hemorrhage referred to as hemophilia.

Of the hemophilias, abnormal hemorrhage due to congenital hypofunction of F.VIII/F.VIIIa or deficiency in F.VIII/F.VIIIa is referred to as hemophilia A. When hemophilia A patient bleeds, replacement therapy with a F.VIII formulation is performed. In addition, preventive administration of a F.VIII formulation may be performed (see Non-Patent Documents 2 and 3) on the day of vigorous exercise or on field trip, when frequent intra-articular bleeding occurs, or when the patient is classified as severe hemophilia. Since this preventive administration of F.VIII formulation remarkably reduces hemorrhage episodes of patients with hemophilia A, it has recently become widely popular. Reduction of bleeding episodes not only reduces lethal and nonlethal bleeding risks and the accompanying agony, but also prevents hemophilic arthropathy caused by frequent intra-articular hemorrhage. As a result, it greatly contributes to the improvement of hemophilia A patients' QOL.

The half life of a F.VIII formulation in blood stream is as short as about 12 to 16 hours. Therefore, for continuous prevention, it is necessary to administer an F.VIII formulation about three times a week. This is equivalent to maintaining approximately 1 % F.VIII activity or more (see Non-Patent Documents 4 and 5). Also, in replacement therapies for bleeding event, it is necessary to periodically administer booster F.VIII formulations for a certain period of time, except when the bleeding is mild, in order to prevent rebleeding and establish complete hemostasis.

Further, F.VIII formulations are intravenously administered. There are technical difficulties in performing intravenous administration, and it becomes even more difficult particularly when performing administration on young patients whose veins are thin.

In the above-described preventive administration of F.VIII formulation and emergency administration thereof in cases of bleeding event, home treatment and self injection are used in most cases. The need for frequent administration and the technical difficulties involved not only inflict pain on patients, but also become a reason that hinders home treatment and self-injection from becoming popular.

Accordingly, there have been strong demands for, as compared to current blood coagulation Factor VIII formulations, drugs that have longer administration intervals and drugs that can be easily administered.

Further, anti-F.VIII antibodies which are referred to as inhibitors may be generated in hemophilia A patients, particularly in severe hemophilia A patients. If an inhibitor is generated, effects of F.VIII formulation become hindered by the inhibitor. As a result, hemostasis control becomes very difficult for patients.

When such hemophilia A inhibitor patient bleeds, neutralization therapy using a mass dose of F.VIII formulation, or bypass therapy using a complex concentrate or F.VIIa formulation is usually performed. However, in neutralization therapy, administration of a mass dose of F.VIII formulation may adversely enhance the inhibitor (anti-F.VIII antibody) titer. Additionally, in bypass therapy, the relatively short half-lives (about 2 to 8 hours) of complex concentrates and the F.VIIa formulation are becoming problematic. Furthermore, since their action mechanisms are independent of the F.VIII/F.VIIIa function, that is, a function to catalyze the activation of F.X by F.IXa, hemostatic mechanism may not function well and become nonresponsive. Therefore, in many cases of hemophilia A inhibitor patients, sufficient hemostatic effects are not obtained when compared to hemophilia A non-inhibitor patients,.

Therefore, there have been strong demands for drugs that are unaffected by the presence of inhibitors and which can functionally substitute for F.VIII/F.VIIIa.

In addition to hemophilia and acquired hemophilia caused by anti-F.VIII autoantibody, von Willebrand's disease, which is caused by functional abnormality or deficiency of vWF, has been known as an abnormal bleeding disorder associated with F.VIII/F.VIIIa. vWF is necessary not only for the normal adhesion of platelets to subendothelial tissues at sites of vessel wall damage, but also for the formation of complexes with F.VIII to maintain a normal plasma F.VIII level. In patients with von Willebrand's disease, these functions decline and functional abnormality of hemostasis occurs.

In the above-described respects, methods that utilize antibodies may be considered for creation of drugs that (i) have long administration intervals, (ii) are easily administered and (iii) are unaffected by the presence of inhibitors, and (iv) can functionally substitute for F.VIII/F.VIIIa in a F.VIII/F.VIIIa-independent manner. Generally, the half-lives of antibodies in blood stream are relatively long - from several days to several weeks. Further, antibodies are known to migrate into the blood stream after subcutaneous administration. That is, antibody drugs meet the above-described requirements of (i) and (ii).

Other embodiments of the present invention's functional proteins include proteins that control multiple different physiological functions by binding to two types of proteins having different physiological functions. Suitable examples include C4b binding protein (C4bp) which binds to fourth component of complement (C4b) and protein S (PS). C4bp not only dissociates C2b from the C4b-C2b complex, but also acts to eliminate the aPC cofactor activity of PS. Therefore, C4bp shows regulatory effects towards the complement system and blood coagulation system. Bispecific antibodies against C4b and PS are thought to have an effect of substituting for the C4bp function. Further, C4bp acts as a cofactor in C4b decomposition by the I Factor. Therefore, bispecific antibodies against the I Factor and C4b are also considered to have an effect of substituting for the C4bp function.

The present invention provides pharmaceutical compositions comprising an antibody of the present invention as an active ingredient. For example, when an antibody of the present invention is an antibody that has an activity of functionally substituting for interferons against cytokine receptors, the antibody is thought to have cytokine-mimetic effects. Therefore, the antibody is expected to become a pharmaceutical (pharmaceutical composition) or drug with an antiviral effect, antitumor effect, and cell growth and/or differentiation regulating effect. On the other hand, an antibody that functionally substitutes for IL-2 is expected to become a pharmaceutical (pharmaceutical composition) or drug with adjuvanticity and/or an anti-tumor effect by differentiation and/or activation of T cells or NK cells; an antibody that functionally substitutes for IL-3 is expected to become a pharmaceutical (pharmaceutical composition) or drug with an effect of promoting hemocyte recovery by proliferation of hemopoietic precursor cells; an antibody that functionally substitutes for IL-4 is expected to become a pharmaceutical (pharmaceutical composition) or drug with an anti-allergic effect by Th2 induction (humoral immunity); an antibody that functionally substitutes for IL-5 is expected to become a pharmaceutical (pharmaceutical composition) or drug with adjuvanticity and/or an anti-tumor effect by B cell induction and/or eosinophil proliferation and/or differentiation; an antibody that functionally substitutes for IL-6 is expected to become a pharmaceutical (pharmaceutical composition) or drug with an effect of stimulating platelet production; an antibody that functionally substitutes for IL-7 is expected to become a pharmaceutical (pharmaceutical composition) or drug with adjuvanticity and/or an anti-tumor effect by proliferation of T cells and/or B cells; an antibody that functionally substitutes for IL-9 is expected to become a pharmaceutical (pharmaceutical composition) or drug with an effect of promoting hemocyte recovery by proliferation and/or hematopoiesis of mast cells; an antibody that functionally substitutes for IL-10 is expected to become a pharmaceutical (pharmaceutical composition) or drug with an immunosuppressive effect; an antibody that functionally substitutes for IL-11 is expected to become a pharmaceutical (pharmaceutical composition) or drug with an effect of stimulating platelet production; an antibody that functionally substitutes for IL-12 is expected to become a pharmaceutical (pharmaceutical composition) or drug with adjuvanticity and/or an anti-tumor effect by Th1 induction (cellular immunity); an antibody that functionally substitutes for IL-15 is expected to become a pharmaceutical (pharmaceutical composition) or drug with adjuvanticity and/or an anti-tumor effect by the activation of NK cells; an antibody that functionally substitutes for GM-CSF is expected to become a pharmaceutical (pharmaceutical composition) or drug with an effect of promoting leukocyte recovery after chemotherapy or bone marrow transplantation; an antibody that functionally substitutes for CNTF is expected to become a pharmaceutical (pharmaceutical composition) or drug with an anti-obesity effect; an antibody that functionally substitutes for LIF is expected to become a pharmaceutical (pharmaceutical composition) or drug with an effect of stimulating platelet production and/or an effect of decreasing blood cholesterol; an antibody that functionally substitutes for Oncostatin M is expected to become a pharmaceutical (pharmaceutical composition) or drug with a hematopoiesis accelerating effect; and an antibody that functionally substitutes for CT-1 is expected to become a pharmaceutical (pharmaceutical composition) or drug with a cardiac muscle protective effect.

Further, when the antibody of the present invention is one of the antibodies that recognize both F.IX or F.IXa and F.X, and can functionally substitute for F.VIIIa, the antibody is expected to become a pharmaceutical (pharmaceutical composition) or drug for preventing or treating bleeding, disorders accompanied by bleeding, or disorders caused by bleeding.

At the same time, it is expected that an antibody that binds to ZPI and F.X and functionally substitutes for PZ becomes a pharmaceutical (pharmaceutical composition) or drug with anti-thrombotic action; an antibody that binds to thrombin and TAFI and functionally substitutes for TM becomes a pharmaceutical (pharmaceutical composition) or drug with hemostasis-promoting action; and a pharmaceutical (pharmaceutical composition) or drug that binds to thrombin and PC and has an effect of functionally substituting for PS/TM system.

In addition, since complement C4 deficiency causes systemic lupus erythematosus (SLE), an antibody that functionally substitutes for C4b is expected to become a pharmaceutical (pharmaceutical composition) or drug with an effect that suppresses SLE occurrence. Since H factor deficiency causes suppurative infection and autoimmune glomerulonephritis, an antibody that functionally substitutes for H factor is expected to become a pharmaceutical (pharmaceutical composition) or drug with an effect of suppressing the onset of these diseases.

Since C4bp deficiency causes Behcet's disease, an antibody that substitutes for the C4bp function is expected to become a pharmaceutical (pharmaceutical composition) or drug with an effect of suppressing the onset of Behcet's disease.

For formulation of pharmaceuticals, pharmaceutical compositions comprising an antibody of the present invention used for treatment or prevention as an active ingredient may be mixed with an appropriate pharmaceutically acceptable carrier, medium and such that are inert thereto, if needed. For example, sterile water or physiological saline, stabilizer, excipient, antioxidant (ascorbic acid etc.), buffer (phosphoric acid, citric acid, other organic acids, etc.), antiseptic, surfactant (PEG, Tween, etc.), chelating agent (EDTA, etc.), binding agent and such can be cited. Pharmaceutical compositions may also contain other low molecular weight polypeptides, proteins such as serum albumin, gelatin, and immunoglobulin, amino acids such as glycine, glutamine, asparagine, arginine, and lysine, sugars such as polysaccharide and monasaccharide and carbohydrates, and sugar alcohols such as mannitol and sorbitol. When preparing aqueous solutions for injection, for example, solubilizing agents include physiological saline, isotonic solutions containing glucose and other adjunctive agents such as D-sorbitol, D-mannose, D-mannitol, and sodium chloride, and may be used in combination with appropriate solubilizing agents such as alcohol (ethanol etc.), polyalcohol (propylene glycol, PEG etc.), and non-ionic surfactant (polysorbate 80, HCO-50 etc.).

Further, if necessary, antibodies of the present invention may be encapsulated into microcapsules (microcapsules made of hydroxymethyl cellulose, gelatin, poly(methyl methacrylate), etc.), or included in a colloidal drug delivery system (liposome, albumin microsphere, microemulsion, nanoparticle, and nanocapsule, etc.) (see "Remington's Pharmaceutical Science 16th edition", Oslo Ed. (1980) etc.). Methods for formulating sustained-release drugs are also known, and can be applied to antibodies of the present invention (Langer *et al.,* J.Biomed.Mater.Res. 15: 267-277 (1981); Langer, Chemtech. 12: 98-105 (1982); U.S. Patent No: 3,773,919; European Patent Application No (EP): 58,481; Sidman *et al.,* Biopolymers 22: 547-556 (1983); EP133,988).

Although the dosage of the pharmaceutical compositions of the present invention is appropriately determined considering the type of formulation, method of administration, age and body weight of patients, symptoms of patients, type and progress of disease, etc, and ultimately by doctors, generally, doses of 0.1-2000 mg/day can be divided into one to several oral administrations for adults. The dosage is preferably 1 to 1000 mg/day, more preferably 5 to 500 mg/day, and most preferably 100 to 300 mg/day. Although the dosage varies according to the body weight and age of patients, administration methods and such, one skilled in the art can suitably select an appropriate dosage. Preferably, the dosing period is also appropriately determined according to, for example, the healing process of patients.

Further, it is also possible to perform gene therapy by inserting a gene encoding an antibody of the present invention into gene therapy vectors. As an administration method apart from direct administration of naked plasmids, the genes may be administered by packaging into liposome and such, or insertion into various virus vectors such as retrovirus vectors, adenovirus vectors, vaccinia virus vectors, pox virus vectors, adeno-associated virus vectors, and HVJ vectors (see Adolph "Virus Genome Method" CRC Press, Florid (1996)), or by coating onto carrier beads such as colloidal gold particle (WO93/17706 etc.). However, the gene may be administered by any methods, as long as the antibody can be expressed *in vivo* to exert its action. Preferably, a sufficient dose is administered through an appropriate parenteral route, such as intravenous, intraperitoneal, subcutaneous, intracutaneous, intra-adipose tissue, intramammary, and intramuscular injection and infusion, inhalation, gas-inducible particle bombardment method (with an electron gun and such), or mucosal route using nasal drop. Genes encoding an antibody of the present invention may be administered by introducing the gene into blood cells, cells derived from bone marrow and such, using *ex vivo* liposome transfection, particle bombardment method (U.S. Patent No. 4,945,050) or virus infection, and re-introducing these cells into animals.

The present invention also provides methods for preventing and/or treating bleeding, disorders accompanied by bleeding, or disorders caused by bleeding, comprising the steps of administering an antibody or composition of this invention. Antibodies or compositions can be administered, for example, by the aforementioned methods.

The present invention also relates to use of the antibodies of this invention for manufacturing (pharmaceutical) compositions of this invention.

Further, the present invention provides kits comprising at least an antibody or composition of this invention to be used in the above-described methods. Glass syringe, injection needle, pharmaceutically acceptable medium, alcohol cotton, bandage, instruction manual that describes the usage, or such may also be optionally packaged into the kits.

### Brief Description of the Drawings

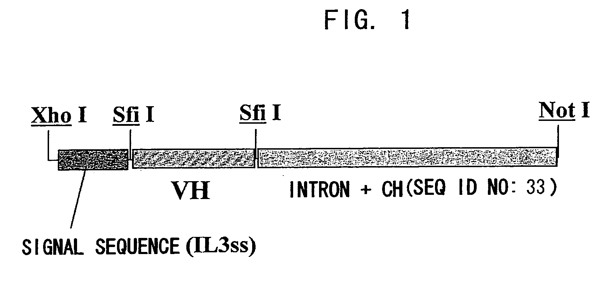

Fig. 1 is a drawing showing the insertion site of pcDNA4-g4H.

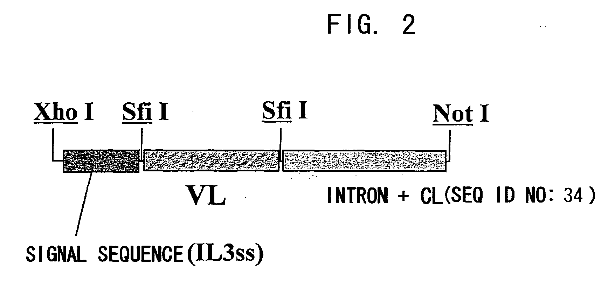

Fig. 2 is a drawing showing the insertion site of pcDNA4-g4L and pIND-g4L.

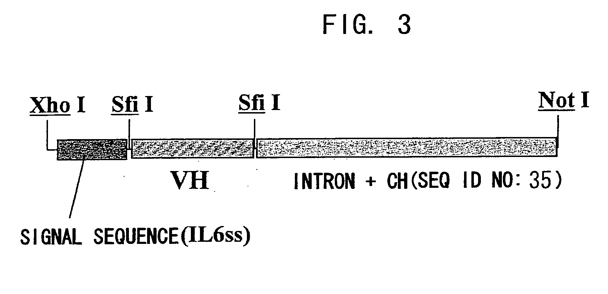

Fig. 3 is a drawing showing the insertion site of pIND-g4H.

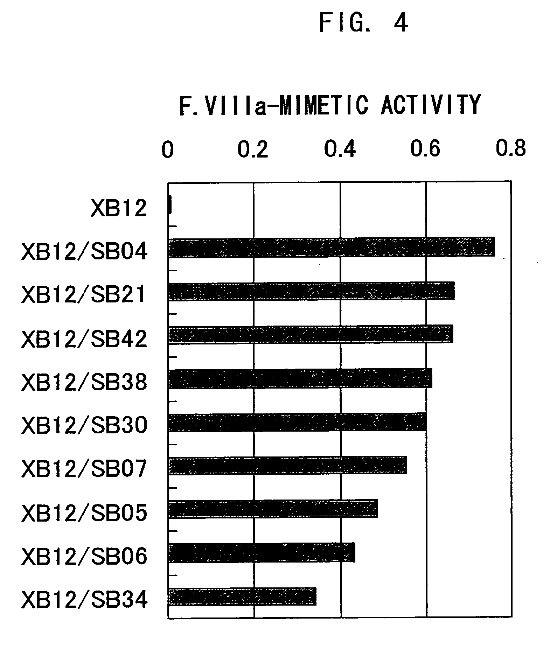

Fig. 4 shows results of measuring the F.VIIIa-mimetic activity of an anti-F.IXa/anti-F.X bispecific antibody generated from anti-F.IXa antibody XB12 and anti-F.X antibody SB04, SB21, SB42, SB38, SB30, SB07, SB05, SB06, or SB34. The concentration of the antibody solutions was 10 µg/mL (final concentration 1 µg/mL). As a result, nine types of bispecific antibodies showed an increase of F.VIIIa-mimetic activity in the order of activity strength: XB12/SB04, XB12/SB21, XB12/SB42, XB12/SB38, XB12/SB30, XB12/SB07, XB12/SB05, XB12/SB06, and XB12/SB34.

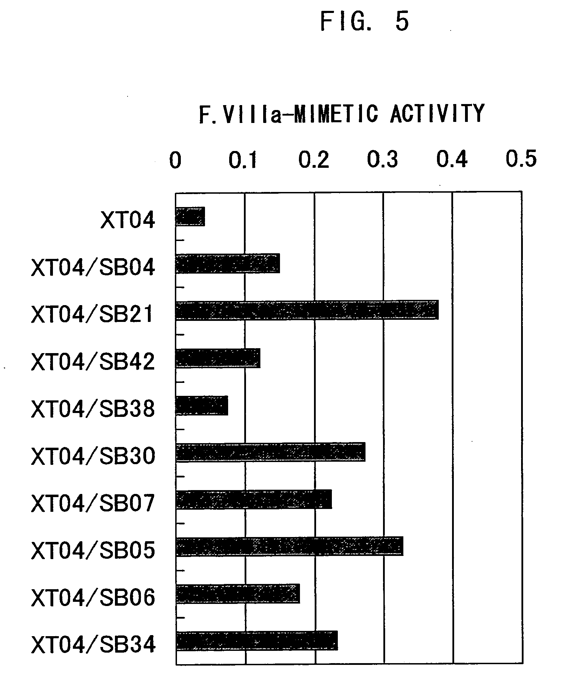

Fig. 5 shows results of measuring the F.VIIIa-mimetic activity of the XT04 antibody or an anti-F.IXa/ F.X bispecific antibody generated from anti-F.IXa antibody XT04 and anti-F.X antibody SB04, SB21, SB42, SB38, SB30, SB07, SB05, SB06, or SB34. The concentration of the antibody solutions was 10 µg/mL (final concentration 1 µg/mL). As a result, XT04/SB04, XT04/SB21, XT04/SB42, XT04/SB38, XT04/SB30, XT04/SB07, XT04/SB05, XT04/SB06, and XT04/SB34 showed elevated F.VIIIa-mimetic activity.

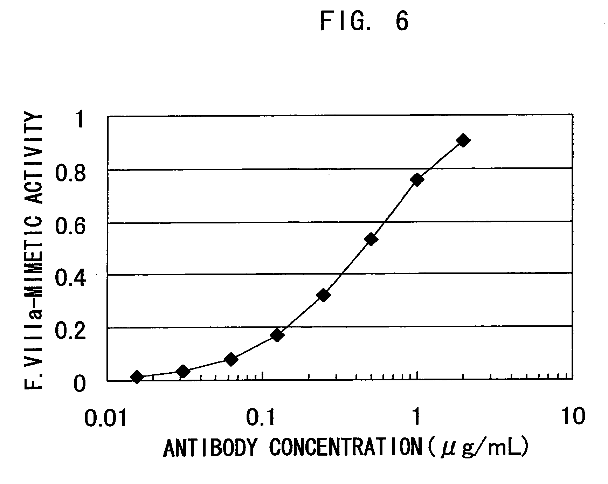

Fig. 6 shows results of measuring the F.VIIIa-mimetic activity of various concentrations of XB12/SB04, which showed the highest activity in Fig. 4. As a result, XB12/SB04 showed a concentration-dependent increase of F.VIIIa-mimetic activity.

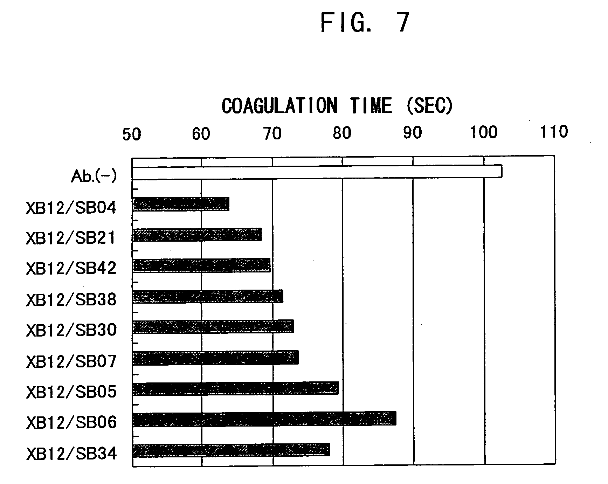

Fig. 7 shows results of measuring the plasma coagulation time (APTT) in the presence of XB12/SB04, XB12/SB21, XB12/SB42, XB12/SB38, XB12/SB30, XB12/SB07, XB12/SB05, XB12/SB06, or XB12/SB34. The antibody solution concentration was 20 µg/mL, except for XB12/SB06 which was 3.4 µg/mL: As a result, XB12/SB04, XB12/SB21, XB12/SB42, XB12/SB38, XB12/SB30, XB12/SB07, XB12/SB05, XB12/SB06, and XB12/SB34 showed a coagulation time shortening effect compared with in the absence of the antibodies.

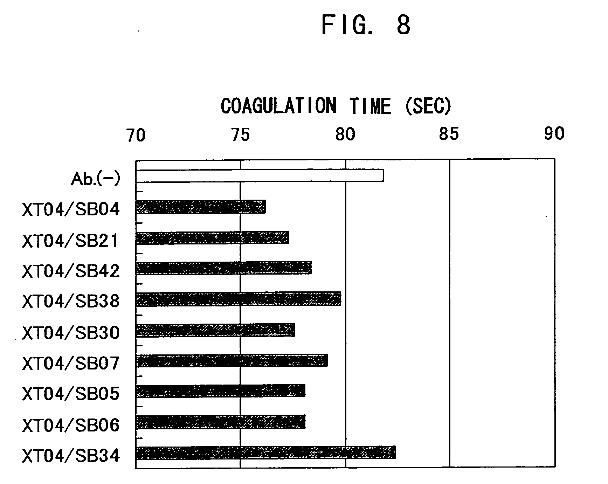

Fig. 8 shows results of measuring the plasma coagulation time (APTT) in the presence of XT04/SB04, XT04/SB21, XT04/SB42, XT04/SB38, XT04/SB30, XT04/SB07, XT04/SB05, XT04/SB06, or XT04/SB34. The antibody solution concentration was 20 µg/mL, except for XT04/SB06 which was 10 µg/mL. As a result, XT04/SB04, XT04/SB21, XT04/SB42, XT04/SB38, XT04/SB30, XT04/SB07, XT04/SB05, and XT04/SB06 showed a coagulation time shortening effect compared with in the absence of the antibodies. XT04/SB34 did not show a coagulation time-shortening effect.

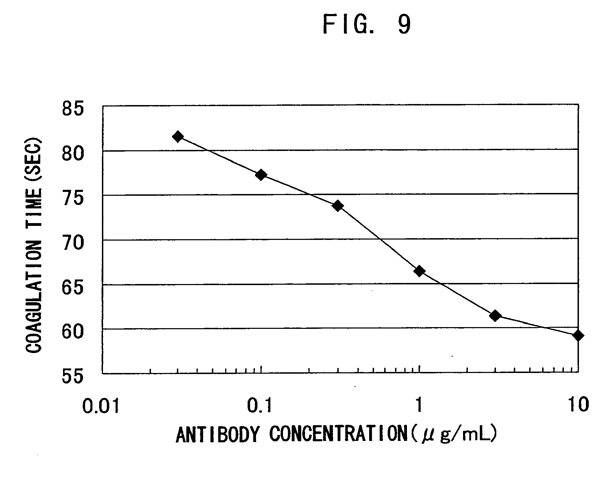

Fig. 9 shows results of measuring the coagulation time with various concentrations of XB12/SB04, which showed the highest coagulation time (APTT)-shortening effect in Figs. 7 and 8. As a result, XB12/SB04 showed a concentration-dependent effect of shortening the coagulation time.

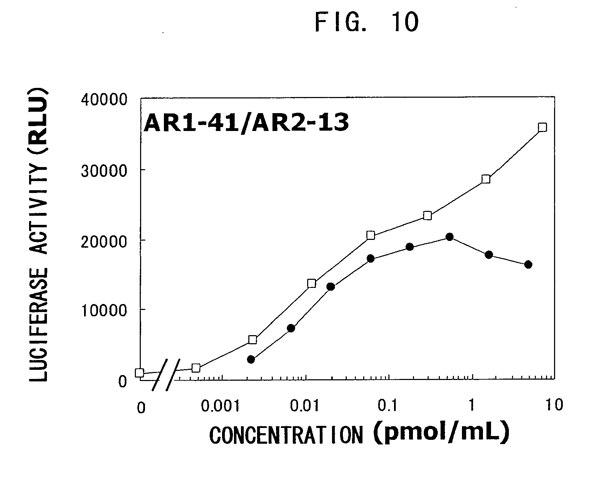

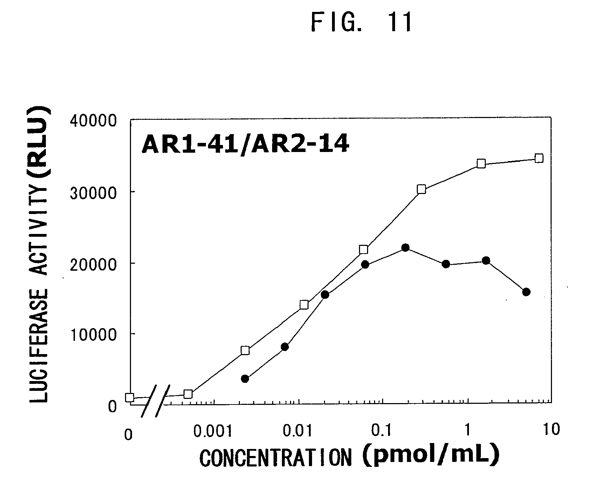

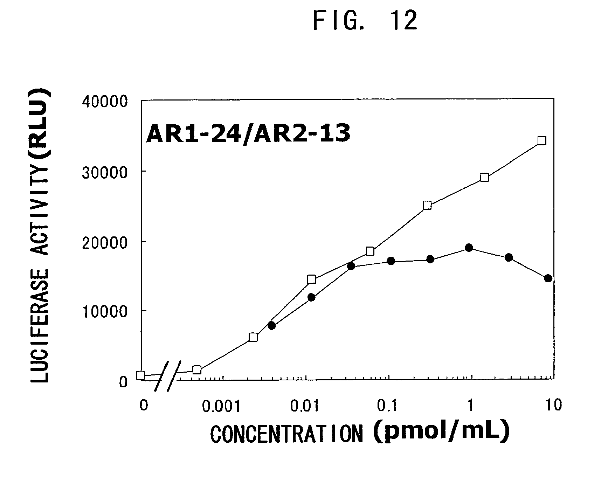

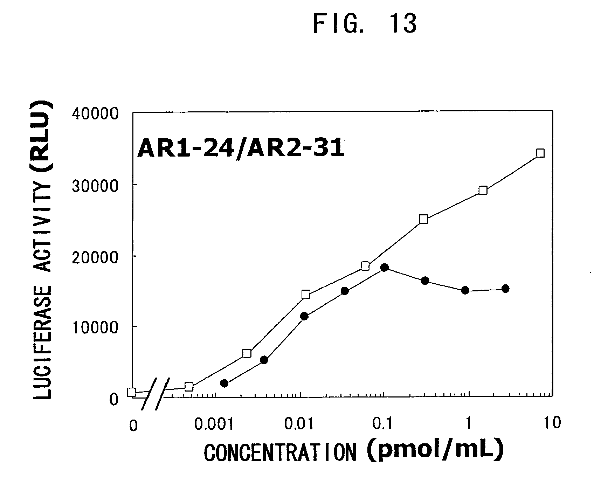

Figs. 10 to 13 show the ISRE activation ability of antibodies against pISRE-Luc introduced K562 cells. □ shows IFN-α2a and • shows the combination of anti-AR1 chain and anti-AR2 chain bispecific antibodies in each figure. The antibodies are shown to activate ISRE in a dose-dependent manner with a per-molecule specific activity comparable to that of IFN.

### Best Mode for Carrying Out the Invention

Herein below, the present invention will be specifically described with reference to Examples, but it is not to be construed as being limited thereto.

### [Example 1] Antigen and immunization

Expression vectors for a soluble receptor, in which the C terminal of the extracellular region of either human AR1 chain or AR2 chain was tagged with FLAG (AR1FLAG, AR2FLAG) or His6 (AR1His, AR2His), were introduced into CHO cells separately and purified from culture supernatants using affinity columns. The expression vector for a chimeric molecule comprising the extracellular region of human AR1 chain and the intracellular region of G-CSF receptor was introduced into mouse proB cell line Ba/F3 to establish a high expression cell line. A high expression cell line was similarly established for a chimeric molecule comprising the extracellular region of human AR2 chain and the intracellular region of G-CSF receptor. The cells were individually used to intraperitoneally immunize BALB/c. AR1 His or AR2His was intravenously injected three days before excising the spleen.

### [Example 2] Separation of antibodies form an scFv presenting library

### (a) Panning of phage library

PolyA(+)RNA was extracted from the spleen of an immunized mouse, and scFv was synthesized by RT-PCR to construct a phagemid library expressing scFv as a fusion protein with gene3 of f1 phage (J. Immun. Methods, 201, (1997), 35-55). The *E. coli* library (2 x 10⁹ cfu) was inoculated into 50 mL of 2x YTAG (2x TY containing 100 µg/mL ampicillin and 2% glucose), and cultured at 37°C till OD 600 reached 0.4 to 0.5. 4 x 10¹¹ of helper phage VCSM13 was added to the culture, which was left to stand at 37°C for 15 minutes for infection. The infected cells were cultured at 26°C for 10 hours, following addition of 450 mL of 2x YTAG and 25 µL of 1 mol/L IPTG. The culture supernatant was collected by centrifugation, mixed with 100 mL of PEG-NaCl (10% polyethylene glycol 8000, 2.5 mol/L NaCl), and left to stand at 4°C for 60 minutes. Phage was precipitated by centrifugation at 10,800x g for 30 minutes, and the precipitate was suspended in 40 mL of water, mixed with 8 mL of PEG-NaCl, and left to stand at 4°C for 20 minutes. Phage was precipitated by centrifugation at 10,800x g for 30 minutes, and suspended in 5 mL of PBS. AR1FLAG and AR2FLAG prepared in Example 1 were labeled with biotin using No-Weigh Premeasured NHS-PEO₄-Biotin Microtubes (Pierce). 100 pmol of biotin labeled AR1FLAG or AR2FLAG was added to the phage library and contacted with the antigen for 60 minutes. 600 µL of Streptavidin MagneSphere (Promega) washed with 5% M-PBS (PBS containing 5% skim milk) added for binding for 15 minutes. Beads were washed with 1 mL PBST (PBS containing 0.1% Tween-20) and PBS three times each. The beads were suspended in 0.8 mL of 0.1 mol/L glycine/HCl (pH 2.2) for 5 minutes to elute the phage. The phage solution thus collected was neutralized by adding 2 mol/L Tris (45 µL), added to 10 mL of XL1-Blue in logarithmic growth phase (OD 600 = 0.4 to 0.5), and left to stand for 30 minutes at 37°C for infection. The mixture was spread on a 2x YTAG plate and cultured at 30°C. Colonies were collected, inoculated into 2x YTAG, and cultured at 37°C until OD 600 = 0.4 to 0.5. IPTG (1 mol/L; 5 µL) and helper phage VCSM13 (10¹¹ pfu) were added to the culture solution (10 mL), and the mixture was left to stand at 37°C for 30 minutes. The cells were collected by centrifugation, resuspended in 2x YTAG (100 mL) containing kanamycin (25 µg/mL), and cultured at 30°C for 10 hours. The culture supernatant was collected by centrifugation, mixed with of PEG-NaCl (20 mL), and left to stand at 4°C for 20 minutes. Phage was precipitated by centrifugation at 10,800x g for 30 minutes, and suspended in PBS (2 mL), and provided for the subsequent panning. Beads were washed five times each for PBST and PBS at the second panning. Clones producing AR chain binding phages were selected by ELISA, from *E.coli* that could infect the eluted phages.

### (b) Phage ELISA

The above-described single colony was inoculated into 2x YTAG (150 µL) and cultured at 30°C overnight. After 5 µL of this culture was inoculated into 2x YTAG (500 µL) and cultured at 37°C for 2 hours, helper phage (2.5 x 10⁹ pfu) and 2x YTAG (100 µL) containing 1 mol/L IPTG (0.3 µL) was added, and the culture was then left to stand at 37°C for 30 minutes. After subsequent overnight culture at 30°C, the centrifuged supernatant was subjected to ELISA. StreptaWell 96 microtiter plate (Roche) was coated over night with PBS (100 µL) containing 1.0 µg/mL biotin-labeled AR1FLAG or AR2FLAG. After washing with PBST to remove the antigen, the reaction was blocked with 200 µL of 2% (w/v) M-PBS over night. After removal of 2% (w/v) M-PBS, the culture supernatant was added therein and left to stand for 40 minutes for antibody binding. After washing, the bound phage was detected with an HRP-bound anti-M13 antibody (Amersham Pharmacia Biotech) diluted with 2% (w/v) M-PBS, and BM blue POD substrate (Roche). The reaction was stopped by adding sulfuric acid, and the A450 value was measured.

### (c) Sequence determination and clone selection

The scFv region was amplified by PCR using primers PBG3-F1 (5'-CAGCTATGAAATACCTATTGCC -3'/ SEQ ID NO: 27) and PBG3-R1 (5'-CTTTTCATAATCAA.AATCACCGG -3'/ SEQ ID NO: 28) from the phage solution of an ELISA positive clone, and its nucleotide sequence was determined. A PCR reaction solution (20 µL) containing 1 µL phage solution, 2 µL 10 x KOD Dash buffer solution, 10 µmol/L primer (0.5 µL each), and 0.3 µL KOD Dash polymerase (TOYOBO, 2.5 U/µL) was amplified on a Perkin Elmer 9700 via 30 cycles of 96°C, 10 seconds, 55°C, 10 seconds, and 72°C, 30 seconds. After PCR, 3 µL of ExoSAP-IT (Amersham) was added to 5 µL of the reaction solution, and incubated at 37°C for 20 minutes, then at 80°C for 15 minutes. This sample was reacted with primer PBG3-F2 (5'- ATTGCCTACGGCAGCCGCT -3'/SEQ ID NO:29) or PBG3-R2 (5'-AAATCACCGGAACCAGAGCC -3'/SEQ ID NO:30) using a BigDye Terminator Cycle Sequencing kit (Applied Biosystems), and electrophoresed on an Applied Biosystems PRISM 3700 DNA Sequencer. For each of the anti-AR1 chain and anti-AR2 chain, 45 clones with CDR3 amino acid sequences different from those predicted from the nucleotide sequences were selected.

### [Example 3] Expression of bispecific antibodies

For expression as scFv-CH1-Fc, an expression vector pCAGGss-g4CH hetero IgG4, where scFv can be inserted between a human signal sequence (driven by promoter CAGG) and the intron -CH1-Fc (human IgG4 cDNA) via an SfiI site, was constructed. For expression as a heteromolecule, amino acid substitutes that are substituted at the CH3 site of IgG4 were produced based on the knobs-into-holes of IgG1 (Ridgway JB *et al.* Protein Engineering 1996, 9: 617-621). Type A is a substitute with Y349C and T366W substitutions, and type B is a substitute with E356C, T366S, L368A, and Y407V substitutions. The substitution of -ppcpScp-to -ppcpPcp- was introduced into the hinge region of both types. Type A was constructed with a human IL-3 signal sequence (pCAGG-IL3ss-g4CHPa) and type B with a human IL-6 signal sequence (pCAGG-IL6ss-g4CHPb). PCR products of the scFv region of the clones selected based on the nucleotide sequences were SfiI treated, then the anti-AR1 chain clone was subcloned into pCAGG-IL3ss-g4CHPa and the anti-AR2 chain clone was subcloned into pCAGG-IL3ss-g4CHPb. Expression vectors for a total of 2025 combinations (anti-AR1 chain and anti-AR2 chain clones 45 x 45) were used to transfect HEK 293 cells using lipofectamine 2000, and three days later, culture supernatants were collected.

### [Example 4] Separation of ligand function-substituting bispecific antibodies

### (a) Ba/F3 growth assay

BaF3-ARG was established by introducing expression vectors for chimeric molecules comprising the extracellular region of AR1 chain or AR2 chain and the intracellular region of G-CSF receptor into Ba/F3 cells, which proliferate in a mouse IL-3-dependent manner. BaF3-ARG proliferated IFNα-dependently. After three washes, 0.1 mL medium containing the sample and 1 x 10³ cells per well was seeded to a 96-well plate. After four days of culture, 10 µL of Cell Count Reagent SF (Nacalai Tesque) was added and incubated at 37°C for two hours, and then A450 was measured.

### (b) Daudi cell proliferation inhibition assay

Daudi cells are a human B cell line with high sensitivity towards IFN. 6.25 x 10³ cells per well were seeded to a 96-well plate in 0.1 mL medium containing the sample. After four days of culture, 10 µL of Cell Count Reagent SF (Nacalai Tesque) was added and incubated at 37°C for two hours, and then A450 was measured.

### (c) Sequences of ligand function-substituting bispecific antibodies

Amino acid sequences of the variable regions of the antibodies selected by the above screening method are described as SEQ ID NOs: 1 to 26. Correlation between the name of each antibody and the SEQ ID NO is shown in the above Table 1.

### (d) Reporter gene assay using ISRE

40 µg of pISRE-Luc was added to 3 mL of OPTI-MEM I and 100 µL DMRIE-C (Invitrogen), stirred, and left to stand at room temperature for 20 minutes. This was added to 8 x 10⁶ human K562 cells prepared in 2 mL OPTI-MEM I, and after four hours of culturing at 37°C, 10 mL of 15%FCS-RPMI1640 was added and the cells were cultured overnight. The next day, K562 collected by centrifugation was resuspended in 10.5 mL of 10% FCS-RPMI1640 and seeded to a 96-well flat bottom plate at 70 µL/well.

Bispecific scFv-CH in the culture supernatants of HEK293 cells introduced with the antibody gene was adjusted to a concentration of 12.5 ng/mL with reference to IgG and a series of 5-fold dilutions were made. Alternatively, culture supernatants of COS7 cells expressing bispecific IgG were diluted 2-fold and a series of 5-fold dilutions were made. These were added to cells introduced with a reporter plasmid at 30 µL/well. For the positive control wells, a series of 5-fold dilutions of IFN-α 2a were dispensed at 30 µL/well. After culturing at 37°C for 24 hours, 50 µL/mL of a Bright-Glo Luciferase Assay System (Promega) was added and left to stand at room temperature for 10 minutes, and luciferase activity was determined with Analyst HT (LJL) (Fig. 10, Fig. 11, Fig. 12, and Fig. 13).

### [Example 5] Preparation of non-neutralizing antibody against Factor IXa (F.IXa)

### 5-1. Immunization and preparation of hybridomas

Eight BALB/c mice (male, 6 weeks old when immunization was initiated (Charles River, Japan)) and five MRL/1pr mice (male, 6 weeks old when immunization was initiated (Charles River, Japan)) were immunized with Factor IXaβ (Enzyme Research Laboratories, Inc.) as described below. As an initial immunization, Factor IXaβ (40 µg/head) emulsified with FCA (Freund's complete adjuvant H37 Ra (Difco laboratories)) was subcutaneously administered. Two weeks later, Factor IXaβ (40 µg/head) emulsified with FIA (Freund's incomplete adjuvant (Difco laboratories)) was subcutaneously administered. Afterward, three to seven booster immunizations were performed at one week intervals. After the titer of a plasma antibody against Factor IXaβ was confirmed to be elevated by ELISA (Enzyme linked immunosorbent assay) described in 5-2, Factor IXaβ (40 µg/head) diluted in PBS(-) (phosphate buffered saline free of calcium ion and magnesium ion) was intravenously administered as a final immunization. Three days after the final immunization, mice spleen cells were fused with mouse myeloma cells P3X63Ag8U.1 (referred to as P3U1, ATCC CRL-1597) by a standard method using PEG1500 (Roche Diagnostics). Fused cells suspended in RPMI1640 medium (Invitrogen) containing 10% FBS (Invitrogen) (hereinafter referred to as 10%FBS/RPMI1640) were seeded in a 96-well culture plate, and 1, 2, 3, and 5 days after the fusion, the medium was replaced with a HAT selection medium (10% FBS/RPMI1640 / 2% HAT 50x concentrate (Dainippon Pharmaceutical Co. Ltd) / 5% BM-Condimed H1 (Roche Diagnostics) to selectively culture hybridomas. Using the supernatants collected on the 8^{th} or 9^{th} day after fusion, Factor IXa-binding activity was measured by ELISA described in 5-2 to select hybridomas having Factor IXa-binding activity. Subsequently, the activity of neutralizing Factor IXa enzymatic activity was measured by the method described in 5-3 to select hybridomas that do not have Factor IXa-neutralizing activity. Hybridomas were cloned twice by performing limiting dilutions in which one cell is seeded in each well of a 96-well culture plate. Single colony cells confirmed by microscopic observation were subjected to ELISA and neutralization activity assay as described in 5-2 and 5-3 was performed for clone selection. Ascites containing the cloned antibody was prepared by the method described in 5-4, and the antibody was purified from the ascites. The purified antibody was unable to extend APTT (activated partial thromboplastin time) and this was confirmed by the method described in 5-5.

### 5-2. Factor IXa ELISA

Factor IXaβ was diluted to 1 µg/mL with a coating buffer (100 mM sodium bicarbonate, pH 9.6, 0.02% sodium azide) and distributed in Nunc-Immuno plate (Nunc-Immuno™ 96 MicroWell™ plates MaxiSorp™ (Nalge Nunc International)) at 100 µL/well. Then, the plate was incubated at 4°C overnight. After washing the plate with PBS(-) containing Tween^{(R)} 20 thrice, it was blocked with a diluent buffer (50 mM Tris-HCl, pH 8.1, 1% bovine serum albumin, 1 mM MgCl₂, 0.15 M NaCl, 0.05% Tween^{(R)} 20, 0.02% sodium azide) at room temperature for 2 hours. After removal of the buffer, a diluent buffer-diluted mouse antiserum or hybridoma culture supernatant was added at 100 µL/well, and incubated at room temperature for 1 hour. After washing the plate thrice, alkaline phosphatase-labeled goat anti-mouse IgG (H+L) (Zymed Laboratories) which had been diluted to 1/2000 with the diluent buffer was added at 100 µL/well, and incubated at room temperature for 1 hour. After washing the plate six times, a colorimetric substrate Blue-Phos™ Phosphate Substrate (Kirkegaard & Perry Laboratories) was added at 100 µL/well, and incubated at room temperature for 20 minutes. After adding the Blue-Phos™ Stop Solution (Kirkegaard & Perry Laboratories) (100 µL/well), absorbance at 595 nm was measured with a Model 3550 Microplate Reader (Bio-Rad Laboratories).

### 5-3. Measurement of Factor IXa neutralizing activity

Phospholipid (Sigma-Aldrich) was dissolved in distilled water for injection, and ultrasonicated to prepare a phospholipid solution (400 µg/mL). Tris buffered saline containing 0.1% bovine serum albumin (hereinafter abbreviated as TBSB) (40 µL), 30 ng/mL Factor IXaβ (Enzyme Research Laboratories) (10 µL), 400 µg/mL phospholipid solution (5 µL), TBSB containing 100 mM CaCl₂ and 20 mM MgCl₂ (5 µL), and hybridoma culture supernatant (10 µL) were mixed in a 96-well plate, and incubated at room temperature for 1 hour. To this mixed solution, 50 µg/mL Factor X (Enzyme Research Laboratories) (20 µL) and 3 U/mL Factor VIII (American diagnostica) (10 µL) were added and reacted at room temperature for 30 minutes. The reaction was stopped by adding 0.5 M EDTA (10 µL). After addition of an S-2222 solution (50 µL; Chromogenix) and incubation at room temperature for 30 minutes, the absorbance was measured at measurement wavelength 405 nm and reference wavelength 655 nm on a Model 3550 Microplate Reader (Bio-Rad Laboratories, Inc.).

### 5-4. Ascites preparation and antibody purification

Ascites of the established hybridomas was produced according to standard procedures. That is, the hybridoma was cultured *in vitro* (2 x 10⁶) and transplanted into the peritoneal cavity of a BALB/c mouse (male, 5 to 7 weeks old at the time experiment was started, Japan Charles River) or BALB/c nude mouse (female, 5 to 6 weeks old at the time experiment was started, Japan Charles River and Japan CLEA), which was intraperitoneally administered twice with pristane (2,6,10,14-tetramethylpentadecane, WAKO Pure Chemical Industries) in advance. One to four weeks after the transplantation, ascites was collected from the mouse with an inflated abdomen.

The antibody was purified from the ascites using a Protein G Sepharose™ 4 Fast Flow column (Amersham Biosciences). The ascites was diluted 2-fold with a binding buffer (20 mM sodium acetate, pH 5.0) and applied to the column, which had been washed with 10 column volumes of the binding buffer. The antibody was eluted with 5 column volumes of an elution buffer (0.1 M glycine-HCl, pH 2.5), and neutralized with a neutralizing buffer (1 M Tris-HCl, pH 9.0). The resulting solution was concentrated using a Centriprep™ 10 (Millipore), and the solvent was replaced with TBS (50 mM Tris-buffered saline). The antibody concentration was calculated from the absorbance at 280 nm with A (1%, 1 cm) = 13.5. Absorbance was measured with DU-650 (Beckman Coulter).

### 5-5. Measurement of APTT (Activated Partial Thromboplastin Time)

APTT was measured with a CR-A (Amelung)-connected KC10A (Amelung). A mixture of the TBSB-diluted antibody solution (50 µL), standard human plasma (Dade Behring) (50 µL), and APTT reagent (Dade Behring) (50 µL) was warmed at 37°C for 3 minutes. To this mixture, 20 mM CaCl₂ (Dade Behring) (50 µL) was added to start a coagulation reaction, and the coagulation time was measured.

### [Example 6] Preparation of non-Factor X (F.X)-neutralizing antibody

### 6-1. Immunization and hybridoma preparation

Eight BALB/c mice (male, 6 weeks old when immunization was initiated, Japan Charles River) and five MRL/1pr mice (male, 6 weeks old when immunization was initiated, Japan Charles River) were immunized with Factor X (Enzyme Research Laboratories) as described below. As an initial immunization, Factor X (40 µg/head) emulsified with FCA was subcutaneously administered. Two weeks later, Factor X (20 or 40 µg/head) emulsified with FIA was subcutaneously administered. Subsequently, three to six booster immunizations were given at one week intervals. After the titer of a plasma antibody against Factor X was confirmed to be elevated by ELISA as described in 6-2, Factor X (20 or 40 µg/head) diluted in PBS (-) was administered intravenously as a final immunization. Three days after the final immunization, mouse spleen cells were fused with mouse myeloma P3U1 cells, according to a standard method using PEG1500. Fused cells suspended in 10% FBS/RPMI1640 medium were seeded in a 96-well culture plate, and hybridomas were selectively cultured by replacing the medium with a HAT selection medium 1, 2, 3 and 5 days after the fusion. Binding activity toward Factor X was measured by ELISA described in 6-2, using the culture supernatant collected on the 8^{th} day after fusion. Hybridomas having Factor X-binding activity were selected, and their activities to neutralize Factor Xa enzymatic activity were measured by the method described in 6-3. Hybridomas that do not have a neutralizing activity toward Factor Xa were cloned by performing limiting dilution twice. Ascites containing the cloned antibody was prepared by the method described in 5-4, and the antibody was purified from the ascites. The purified antibody was unable to extend APTT and this was confirmed by the method described in 5-5.

### 6-2. Factor X ELISA

Factor X was diluted to 1 µg/mL with a coating buffer, and dispersed into Nunc-Immuno plate at 100 µL/well. Then the plate was incubated at 4°C overnight. After washing the plate with PBS (-) containing Tween^{(R)} 20 thrice, it was blocked with a diluent buffer at room temperature for 2 hours. After removal of the buffer, a diluent buffer-diluted mouse antiserum or hybridoma culture supernatant was added to the plate, and incubated at room temperature for 1 hour. After washing the plate thrice, alkaline phosphatase-labeled goat anti-mouse IgG (H+L) which had been diluted to 1/2000 with the diluent buffer was added, and incubated at room temperature for 1 hour. After washing the plate six times, a colorimetric substrate Blue-Phos™ Phosphate Substrate (Kirkegaard & Perry Laboratories)was added at 100 µL/well, and incubated at room temperature for 20 minutes. After adding Blue-Phos™ Stop Solution (Kirkegaard & Perry Laboratories) (100 µL/well), absorbance ate 595 nm was measured with a Model 3550 Microplate Reader (Bio-Rad Laboratories).

### 6-3. Measurement of Factor Xa-neutralizing activity

Hybridoma culture supernatant diluted to 1/5 with TBSB (10 µL) was mixed with 40 µL of TBCP (TBSB containing 2.78 mM CaCl₂ and 22.2 µM phospholipids (phosphatidyl choline:phosphatidyl serine = 75:25, Sigma-Aldrich) containing 250 pg/mL Factor Xa (Enzyme Research Laboratories), and incubated at room temperature for 1 hour. To this mixed solution, TBCP (50 µL) containing prothrombin (Enzyme Research Laboratories) (20 µg/mL) and 100 ng/mL activated coagulation factor V (Factor Va (Haematologic Technologies)) were added, and reacted at room temperature for 10 minutes. The reaction was stopped by adding 0.5 M EDTA (10 µL). To this reaction solution, 1 mM S-2238 solution (Chromogenix) (50 µL) was added, and after incubation at room temperature for 30 minutes, absorbance at 405 nm was measured with a Model 3550 Microplate Reader (Bio-Rad Laboratories).

### [Example 7] Construction of chimera bispecific antibody expression vector

### 7-1. Preparation of antibody variable region-encoding DNA fragments from hybridomas

From the hybridomas that produce anti-F.IXa antibody or anti-F.X antibody, total RNA was extracted using the QIAGEN^{(R)} RNeasy^{(R)} Mini Kit (QIAGEN) according to the method described in the instruction manual. The total RNA was dissolved in sterile water (40 µL). Single-stranded cDNA was synthesized by RT-PCR using the SuperScript cDNA synthesis system (Invitrogen) with the purified RNA (1 to 2 µg) as template, according to the method described in the instruction manual.

### 7-2. PCR amplification of antibody H chain variable region and sequence analysis

As primers for amplifying the mouse antibody H chain variable region (VH) cDNA, an HB primer mixture and HF primer mixture described in the report by Krebber *et al.* (J. Immunol. Methods 1997; 201: 35-55) were prepared. Using 0.5 µL each of the 100 µM HB primer mixture and 100 µM HF primer mixture, a reaction solution (25 µL) (cDNA solution prepared in 7-1 (2.5 µL), KOD plus buffer (TOYOBO), 0.2 mM dNTPs, 1.5 mM MgCl₂, 0.75 units DNA polymerase KOD plus (TOYOBO)) was prepared. Using a thermal cycler GeneAmp PCR system 9700 (Parkin Elmer), PCR was performed according to amplification efficiency of the cDNA fragments, either under conditions A (3 min heating at 98°C followed by 32 cycles of reaction (98°C, 20 sec, 58°C, 20 sec, and 72°C, 30 sec in one cycle)) or conditions B (3 min heating at 94°C followed by 5 cycles of reaction (94°C, 20 sec, 46°C, 20 sec, and 68°C, 30 sec in one cycle) and 30 cycles of reaction (94°C, 20 sec, 58°C, 20 sec, and 72°C, 30 sec in one cycle)). After PCR, the reaction solution was subjected to 1% agarose gel electrophoresis. Amplified fragments of the desired size (about 400 bp) were purified using a QIAquick Gel Extraction Kit (QIAGEN) according to the methods described in the attached instruction manual, and eluted with sterile water (30 µL). Nucleotide sequences of the DNA fragments were determined using a BigDye Terminator Cycle Sequencing Kit (Applied Biosystems) on a DNA sequencer ABI PRISM 3100 Genetic Analyzer (Applied Biosystems), according to the method described in the attached instruction manual. Sequence groups determined by this method were comparatively analyzed using an analytical software, GENETYX-SV/RC Version 6.1 (Genetyx), and DNAs with different sequences were selected.

### 7-3. Preparation of antibody variable region DNA fragments for cloning

The following procedure was performed to add restriction enzyme Sfi I cleavage sites for cloning to both termini of the antibody variable region amplification fragments.

To amplify the VH fragments added with an Sfi I cleavage site (Sfi I-VH), a primer (primer VH-5' end) in which the primer HB (Gly4Ser)2-linker sequence was replaced with a sequence containing Sfi I cleavage site (SEQ ID NO: 31) was prepared. Using 0.5 µL each of the 10 µM sequence-specific primer VH-5' end and 10 µM primer scfor (J. Immunol. Methods 1997; 201: 35-55), a reaction solution (20 µL) (purified solution of VH cDNA amplification fragment prepared in 7-2 (1 µL), KOD plus buffer (TOYOBO), 0.2 mM dNTPs, 1.5 mM MgCl₂, 0.5 units DNA polymerase KOD plus (TOYOBO)) was prepared. Using a thermal cycler GeneAmp PCR system 9700 (Parkin Elmer), PCR was performed according to amplification efficiency of the cDNA fragments, either under conditions A (3 min heating at 98°C followed by 32 cycles of reaction (98°C, 20 sec, 58°C, 20 sec, and 72°C, 30 sec in one cycle)) or conditions B (3 min heating at 94°C followed by 5 cycles of reaction (94°C, 20 sec, 46°C, 20 sec, and 68°C, 30 sec in one cycle) and 30 cycles of reaction (94°C, 20 sec, 58°C, 20 sec, and 72°C, 30 sec in one cycle)). After PCR, the reaction solution was subjected to 1% agarose gel electrophoresis. Amplified fragments of the desired size (about 400 bp) were purified using a QIAquick Gel Extraction Kit (QIAGEN) by the method described in the attached instruction manual, and eluted with sterile water (30 µL).

To amplify the mouse antibody L chain variable region (VL) cDNA fragments, 0.5 µL each of the 100 µM LB primer mixture and 100 µM LF primer mixture described in the report by Krebber *et al.* (J. Immunol. Methods 1997; 201: 35-55) was first used, and a reaction solution (25 µL) (cDNA solution prepared in 7-1 (2.5 µL), KOD plus buffer (TOYOBO), 0.2 mM dNTPs, 1.5 mM MgCl₂, 0.75 units DNA polymerase KOD plus (TOYOBO)) was prepared. Using a thermal cycler GeneAmp PCR system 9700 (Parkin Elmer), PCR was performed according to amplification efficiency of the fragments, under conditions of 3 minutes heating at 94°C followed by 5 cycles of reaction (94°C, 20 sec, 46°C, 20 sec, and 68°C, 30 sec in one cycle) and 30 cycles of reaction (94°C, 20 sec, 58°C, 20 sec, and 72°C, 30 sec in one cycle). After PCR, the reaction solution was subjected to 1% agarose gel electrophoresis. Amplified fragments of the desired size (about 400 bp) were purified using the QIAquick Gel Extraction Kit (QIAGEN) by the method described in the attached instruction manual, and eluted with sterile water (30 µL). The fragments are in a state in which the primer LF-derived (Gly4Ser)3-linker sequence is added to their C termini. In order to add an Sfi I cleavage site to the C termini of the fragments, a primer (primer VL-3' end) in which the primer LF (Gly4Ser)3-linker sequence was replaced with a sequence having Sfi I cleavage site (SEQ ID NO: 32) was prepared. To amplify the VL fragments added with an Sfi I cleavage site (Sfi I-VL), 0.5 µL each of the 10 µM VL-3' end primer mixture and 10 µM scback primer was used, and a reaction mixture (20 µL) (purified solution of VL cDNA amplification fragment (1 µL), KOD plus buffer (TOYOBO), 0.2 mM dNTPs, 1.5 mM MgCl₂, 0.5 units DNA polymerase KOD plus (TOYOBO)) was prepared. PCR was performed using a thermal cycler GeneAmp PCR system 9700 (Parkin Elmer) under conditions of 3-minutes heating at 94°C followed by 5 cycles of reaction (94°C, 20 sec, 46°C, 20 sec, and 68°C, 30 sec in one cycle) and 30 cycles of reaction (94°C, 20 sec, 58°C, 20 sec, and 72°C, 30 sec in one cycle). After PCR, the reaction solution was subjected to 1% agarose gel electrophoresis. Amplified fragments of the desired size (about 400 bp) were purified using the QIAquick Gel Extraction Kit (QIAGEN) by the method described in the attached instruction manual, and eluted with sterile water (30 µL).

The purified Sfi I-VH and Sfi I-VL fragments were digested with Sfi I (Takara Bio) at 50°C overnight in a reaction solution prepared according to the method described in the attached instruction manual. Subsequently, the reaction solution was purified using a QIAquick PCR Purification Kit (QIAGEN) by the method described in the attached instruction manual, and eluted with Buffer EB (30 µL) included in the kit.

### 7-4. Human IgG4-Mouse chimera bispecific IgG antibody expression plasmid

When producing the bispecific IgG antibody of interest, the knobs-into-holes technique of IgG1 (Ridgway *et al.,* Protein Eng. 1996; 9: 617-621) was referred to when preparing IgG4 with an amino acid-substituted CH3 portion to form heteromolecules for each H chain. Type a (IgG4γa) is substituted with Y349C and T366W, and type b (IgG4γb) is substituted with E356C, T366S, L368A, and Y407V. Further, a substitution (-ppcpScp- -> -ppcpPcp-) was also introduced at the hinge regions of both types. Almost all the H chains become heteromolecules by this technique; however, this does not necessarily apply to L chains, and the formation of unnecessary antibody molecules may affect subsequent activity measurements. Therefore, to separately express the arms of each antibody molecule (called HL molecule), which have different specificities, and efficiently form the type of bispecific IgG antibody of interest within cells, those that are inducible by different drugs were used as the expression vectors for each HL molecule.

As an expression vector for an arm of the antibody molecule (called right arm HL molecule for convenience), pcDNA4-g4H or pcDNA4-g4L (Fig. 1 or Fig. 2) was prepared, in which the respective H chain or L chain region, that is, an appropriate mouse antibody variable region (VH or VL) and a human IgG4γa constant region (SEQ ID NO: 33) or κ constant region (SEQ ID NO: 34), were incorporated into the tetracycline-inducible type vector pcDNA4 (Invitrogen) downstream of the signal sequence (IL3ss) for animal cells (Proc. Natl. Acad. Sci. USA. 1984; 81: 1075). First, Eco RV and Not I (Takara Bio) were used to digest pcDNA4 at the restriction enzyme cleavage sites that are present in its multi-cloning site. The right arm H chain- or L chain-expression unit (about 1.6 kb or about 1.0 kb respectively) of a chimera bispecific antibody having appropriate antibody variable regions was digested with Xho I (Takara Bio). Then, it was purified with the QIAquick PCR Purification Kit (QIAGEN) by the method described in the attached instruction manual, and reacted with DNA polymerase KOD (TOYOBO) at 72°C for 10 minutes in a reaction solution composition described in the attached instruction manual to blunt the ends. The blunt-end fragments were purified with the QIAquick PCR Purification Kit (QIAGEN) by the method described in the attached instruction manual, and digested with Not I (Takara Bio). The Not I/blunt end fragments (about 1.6 kb or 1.0 kb respectively) and the Eco RV/Not I-digested pcDNA4 were subjected to a ligation reaction using Ligation High (TOYOBO), according to the method described in the attached instruction manual. An *E. coli* DH5α strain (Competent high DH5α (TOYOBO)) was transformed with the above-described reaction solution. From the ampicillin-resistant clones thus obtained, respective plasmid DNAs were isolated using the QIAprep Spin Miniprep Kit (QIAGEN).

As an expression vector for the antibody molecule's other arm (called left arm HL molecule for convenience), pIND-g4H or pIND-g4L (Fig. 2 or Fig. 3) was prepared according to the above-described method, in which the H chain or L chain respective region, that is, an appropriate mouse antibody variable region (VH or VL) and a human IgG4γb constant region (SEQ ID NO: 35) or κ constant region (SEQ ID NO: 34), were incorporated into the ecdysone analogue inducible type vector pIND (Invitrogen) downstream of the signal sequence (IL3ss) for animal cells (EMBO. J. 1987; 6: 2939), and the respective plasmid DNAs were isolated.

### 7-5. Construction of bispecific antibody expression vector

The tetracycline-inducible type expression plasmid prepared in 7-4 (pcDNA4-g4H or pcDNA4-g4L) was digested with Sfi I, and was subjected to 1% agarose gel electrophoresis. Fragments (about 5 kb) lacking the intrinsic antibody variable region part (VH or VL (see Fig. 1 or Fig. 2)) were purified using the QIAquick Gel Extraction Kit (QIAGEN) by the method described in the attached instruction manual, and eluted with sterile water (30 µL). The fragments, and the corresponding Sfi I-VH or Sfi-VL fragment derived from the Sfi I-digested anti-F.IXa antibody prepared in 7-3, were subjected to a ligation reaction using the Quick Ligation Kit (New England Biolabs) according to the method described in the attached instruction manual. An *E. coli* DH5α strain (Competent high DH5α (TOYOBO)) was transformed with the above-described reaction solution. Further, fragments obtained by removing the antibody variable region part by a similar technique as described above (VH or VL (see Fig. 2 or Fig. 33)) from the Sfi I-digested ecdysone analogue-inducible type expression plasmid (pIND-g4H or pIND-4GL) prepared in 7-4 and the corresponding Sfi I-digested anti-F.X antibody-derived Sfi I-VH or Sfi I-VL fragment prepared in 7-3 were incorporated by a similar method.

In each of the ampicillin-resistant transformants thus obtained, insertion of the fragment of interest was confirmed by colony PCR method using primers that sandwich the inserted fragment. First, for the anti-F.IXa antibody chimeric H chain or L chain expression vector, a 21-mer CMVF primer (SEQ ID NO: 36) which anneals to the CMV forward priming site upstream of the insertion site, and an 18-mer BGHR primer (SEQ ID NO: 37) which anneals to the BGH reverse priming site downstream of the insertion site were synthesized (Sigma Genosys). For the anti-F.X antibody chimeric H chain or L chain expression vector, a 24-mer EcdF primer (SEQ ID NO: 38), which anneals to the upstream of the insertion site and an 18-mer BGHR primer (SEQ ID NO: 37) which anneals to the BGH reverse priming site downstream of the insertion site were synthesized (Sigma Genosys). For colony PCR, a reaction solution (20 µL) (0.2 µL primer (10 µM), KOD dash buffer (TOYOBO), 0.2 mM dNTPs, and 0.75 units DNA polymerase KOD dash) (TOYOBO)) was prepared. To this reaction solution, cells of the transformant strain were added in appropriate amounts and PCR was performed. PCR was performed using a thermal cycler GeneAmp PCR system 9700 (Parkin Elmer) under conditions of 1 minute heating at 96°C followed by 30 cycles of reaction (96°C, 10 sec, 55°C, 10 sec, and 72°C, 30 sec in one cycle). After PCR, the reaction solution was subjected to 1% agarose gel electrophoresis, and clones from which amplification fragments of the desired size were obtained were selected. The PCR product was treated with an ExoSAP-IT (Amersham Biosciences) to inactivate excess primers and dNTPs according to the attached instruction manual. Nucleotide sequences of the DNA fragments were determined using a BigDye Terminator Cycle Sequencing Kit (Applied Biosystems) on a DNA sequencer ABI PRISM 3100 Genetic Analyzer (Applied Biosystems), according to the method described in the attached instruction manual. Sequence groups determined by the present method were analyzed with an analytical software, GENETYX-SV/RC Version 6.1 (Genetyx). For VH, clones of interest having no insertion, deletion, or mutation were selected. For VL, different from the P3U1-derived pseudo VL gene used in hybridomas, clones of interest having no insertion, deletion, or mutation were selected.

From the clones of interest, the respective plasmid DNAs were isolated by using a QIAprep Spin Miniprep Kit (QIAGEN), and then dissolved in sterile water (100 µL). Anti-F.IXa antibody chimeric H chain expression vector, anti-F.IXa antibody chimeric L chain expression vector, anti-F.X antibody chimeric H chain expression vector, and anti-F.X antibody chimeric L chain expression vector were named pcDNA4-g4IXaHn, pcDNA4-g4IXaLn, pIND-g4XHn, and pIND-g4XLn, respectively. Each plasmid solution was stored at 4°C till use.

### [Example 8] Expression of chimera bispecific antibodies in animal cells

### 8-1. Preparation of DNA solutions

Expression of the antibody's right arm HL molecule expression vectors (pcDNA4-g4IXaHn and pcDNA4-g4IXaLn) is induced by tetracycline. In the absence of tetracycline, Tet repressor-encoding plasmid pcDNA6/TR (Invitrogen) is required to completely suppress their expressions. Further, expression of the left arm antibody HL molecule expression vectors (pIND-g4XHn and pIND-g4XLn) was induced by an insect hormone ecdysone analogue (ponasterone A). This requires plasmid pVgRXR (Invitrogen) which encodes the ecdysone receptor and retinoid X receptor that react with ponasterone A and induce expression. Therefore, for the transfection of animal cells, a mixture of six types of plasmid DNAs in total was prepared. For 1 mL of cell culture, pcDNA4-g4IXaHn, pcDNA4-g4IXaLn, pIND-g4XHn and pIND-g4XLn (218.8 ng each), as well as pcDNA6/TR and pVgRXR (1312.5 ng each) were used.

### 8-2 Transfection of animal cells

A HEK293H strain (Invitrogen) derived from human fetal renal cancer cells was suspended in DMEM medium (Invitrogen) containing 10% FCS (MOREGATE), plated onto each well of a 12-well plate for cell adhesion at a cell density of 5 x 10⁵ cells/mL, and cultured in a CO₂ incubator (37°C, 5% CO₂), The plasmid DNA mixture prepared in 8-1 was added to a mixed solution of transfection reagent Lipofectamine 2000 (7 µL; Invitrogen) and Opti-MEM I medium (250 µL; Invitrogen) and left to stand at room temperature for 20 minutes. This mixed solution was added to cells in each well, and the cells were incubated in a CO₂ incubator (37°C, 5% CO₂ for four to five hours.

### 8-3 Induction of bispecific IgG antibody expression

After the medium was removed by suction from the above transfected cell cultures, 1 mL CHO-S-SFM-II (Invitrogen) medium containing 1 µg/mL tetracycline (WAKO Pure Chemical Industries) was added, and primary expression of the antibody's right arm HL molecule was induced by culturing the cells in a CO₂ incubator (37°C, 5% CO₂) for one day. Subsequently, the medium was removed by suction, and the cells were washed once with 1 mL of CHO-S-SFM-II medium, and cultured in a CO₂ incubator (37°C, 5% CO₂ for 2 or 3 days following the addition of 1 mL of CHO-S-SFM-II medium containing 5 µM ponasterone A (Invitrogen), and secondary expression of the antibody's left arm HL molecule was induced for secretion of the bispecific IgG antibody into the medium. The collected culture supernatant was centrifuged (approximately 2000g, 5min, room temperature) to remove the cells, and concentrated as needed by Microcon^{(R)} YM-50 (Millipore). The samples were stored at 4°C till use.

### [Example 9] Quantification of human IgG concentration

Goat affinity purified antibody to human IgG Fc (Cappel) was prepared at 1 µg/mL with a coating buffer, and solid-phased onto a Nunc-Immuno plate. After blocking with a diluent buffer (D.B.), samples of culture supernatants appropriately diluted with D.B. were added. As a standard for calculating the antibody concentration, human IgG4 (humanized anti-TF antibody, see WO 99/51743) diluted with D.B. in a 2-fold dilution series with 11 levels from 1000 ng/mL was similarly added. After three washes, alkaline phosphatase goat anti-human IgG (Biosource International) was added for reaction. After five washes, the plate was color developed using the Sigma 104^{(R)} phosphatase substrate (Sigma-Aldrich) as a substrate, and the absorbance at 405 nm was measured on an absorbance reader Model 3550 (Bio-Rad Laboratories) with a reference wavelength of 655 nm. Using the Microplate Manager III (Bio-Rad Laboratories) software, human IgG concentration in the culture supernatant was calculated from the standard curve.

### [Example 10] F.VIIIa (activated coagulation factor VIII)-mimetic activity assay

The F.VIIIa-mimetic activity of a bispecific antibody was assessed by the following enzymatic assay. The following reactions were all performed at room temperature. A mixed solution of 40 µL Factor IX (3.75 µg/mL; Enzyme Research Laboratories) and 10 µL of the antibody solution was incubated in a 96-well plate for one hour. Then, 10 µL Factor XIa (10 ng/mL; Enzyme Research Laboratories), 20 µL Factor X (50 µg/mL; Enzyme Research Laboratories), 5 µL phospholipid (400 µg/mL; see Example 5-3), and 15 µL TBSB containing 5mM CaCl₂ and 1mM MgCl₂ (hereinafter abbreviated as TBSB-S) were added to initiate the enzymatic reaction. After one hour, the reaction was stopped by adding 10 µL of 0.5M EDTA.

After adding a colorimetric substrate solution (50 µL) to each well, absorbance at 405 nm (reference wave length 655 nm) was measured at 0 and 30 minutes with a Model 3550 Microplate Reader (Bio Rad Laboratories). The F.VIIIa-mimetic activity was presented as a value obtained by subtracting the value of absorbance change in 30 minutes without antibody addition from that with the antibody addition (see Fig. 4 and Fig. 5).

TBSB was used as a solvent for phospholipids, while TBSB-S was used as a solvent for Factor XIa, Factor IX, and Factor X. The colorimetric substrate solution was a 1:1 mixture of "Tesutochimu" colorimetric substrate S-2222 (Chromogenix) dissolved according to the attached instruction manual and a polybrene solution (0.6 mg/L hexadimethrine bromide (Sigma)).

Further, the concentration dependency of XB12/SB04's F.VIIIa-mimetic activity, which was the highest among all, was measured (Fig. 6).

### [Example 11] Plasma coagulation assay

To elucidate whether a bispecific antibody corrects the coagulation ability of hemophilia A blood, effects of the bispecific antibody on activated partial thromboplastin time (APTT) were examined using F.VIII-deficient plasma. A mixed solution comprising an antibody solution at various concentrations (50 µL), F.VIII-deficient plasma (50 µL; Biomerieux) and APTT reagent (50 µL; Dade Behring) was warmed at 37°C for 3 minutes. Coagulation reaction was initiated by adding 20 mM CaCl₂ (50 µL; Dade Behring) to the above-described mixture. The time required for coagulation was measured with CR-A (Amelung)-connected KC10A (Amelung) (Figs. 7 and 8).

Further, XB12/SB04, which showed the highest coagulation time-shortening activity, was measured for its concentration dependency (Fig. 9).

### [Example 12] Antibody purification

The culture supernatant (10 mL) obtained by the method described in Example 8 was concentrated to 1 mL with Centricon^{(R)} YM-50 (Millipore). To this concentrate, 10% BSA (10 µL), 1% Tween^{(R)} 20 (10 µL), and rProtein A Sepharose™ Fast Flow (Amersham Biosciences) (100 µL) were added, and the solution was mixed by overturning at 4°C overnight. The solution was transferred to an Ultrafree^{(R)}-MC 0.22 µm filter cup (Millipore), and after washing with TBS containing 0.01% Tween^{(R)} 20 (500 µL) thrice, the rProtein A Sepharose™ resin was suspended in 100 µL of 0.01% Tween^{(R)} 20 (pH 2.0) containing 10 mM HCl, and left to stand for 3 minutes. Then, the antibody was eluted, and the eluate was immediately neutralized with the addition of 5 µL 1M Tris-HCl, pH 8.0. Using the Microplate Manager III (Bio-Rad Laboratories) software, the human IgG concentration was calculated from the standard curve. The antibody concentration was quantified according to Example 9.

### Industrial Applicability

The present invention provides bispecific antibodies that have the effect of functionally substituting for ligands of heteromolecule-comprising receptors.

The present invention also provides bispecific antibodies that recognize both an enzyme and its substrate, and which functionally substitute for a cofactor that enhances the enzymatic activity.

The bispecific antibodies according to the present invention are thought to have high stability in blood and low antigenicity. Thus, it is greatly expected that they will become pharmaceuticals.

## Claims

1. A bispecific antibody that substitutes for the effect of a functional protein.

2. A bispecific antibody that has an activity of functionally substituting for a ligand of a heteromolecule-comprising receptor.

3. The antibody according to claim 2, wherein said heteromolecule-comprising receptor is a dimer.

4. The antibody according to claim 2, wherein said receptor is a cytokine receptor.

5. The antibody according to claim 4, wherein said cytokine receptor is an interferon receptor.

6. The antibody according to claim 5, wherein said interferon receptor is a type I interferon receptor.

7. The antibody according to claim 6, wherein said type I interferon receptor comprises an AR1 chain and an AR2 chain.

8. The antibody according to claim 7, wherein said antibody functionally substitutes for an interferon which is a ligand of a type I interferon receptor.

9. The antibody according to claim 8, wherein said antibody comprises the variable region of an anti-AR1 chain antibody and the variable region of an anti-AR2 chain antibody.

10. The antibody according to claim 9, wherein said antibody comprises an anti-AR1 chain antibody variable region comprising the amino acid sequence of (a) below and an anti-AR2 chain antibody variable region comprising the amino acid sequence of any of the following (b1) to (b10):
(a) the H chain variable region amino acid sequence described in SEQ ID NO: 1 and the L chain variable region amino acid sequence described in SEQ ID NO:2;
(b1) the H chain variable region amino acid sequence described in SEQ ID NO: 7 and the L chain variable region amino acid sequence described in SEQ ID NO: 8;
(b2) the H chain variable region amino acid sequence described in SEQ ID NO: 9 and the L chain variable region amino acid sequence described in SEQ ID NO: 10;
(b3) the H chain variable region amino acid sequence described in SEQ ID NO: 19 and the L chain variable region amino acid sequence described in SEQ ID NO: 20;
(b4) the H chain variable region amino acid sequence described in SEQ ID NO: 13 and the L chain variable region amino acid sequence described in SEQ ID NO: 14;
(b5) the H chain variable region amino acid sequence described in SEQ ID NO: 23 and the L chain variable region amino acid sequence described in SEQ ID NO: 24;
(b6) the H chain variable region amino acid sequence described in SEQ ID NO: 5 and the L chain variable region amino acid sequence described in SEQ ID NO: 6;
(b7) the H chain variable region amino acid sequence described in SEQ ID NO: 17 and the L chain variable region amino acid sequence described in SEQ ID NO: 18;
(b8) the H chain variable region amino acid sequence described in SEQ ID NO: 15 and the L chain variable region amino acid sequence described in SEQ ID NO: 16;
(b9) the H chain variable region amino acid sequence described in SEQ ID NO: 21 and the L chain variable region amino acid sequence described in SEQ ID NO: 22;
(b10) the H chain variable region amino acid sequence described in SEQ ID NO: 11 and the L chain variable region amino acid sequence described in SEQ ID NO: 12.

11. The antibody according to claim 9, wherein said antibody comprises an anti-AR1 chain antibody variable region comprising the amino acid sequence of (a) below or an anti-AR2 chain antibody variable region comprising the amino acid sequence of any of the following (b1) to (b3):
(a) the H chain variable region amino acid sequence described in SEQ ID NO: 3 and the L chain variable region amino acid sequence described in SEQ ID NO: 4;
(b1) the H chain variable region amino acid sequence described in SEQ ID NO: 25 and the L chain variable region amino acid sequence described in SEQ ID NO: 26;
(b2) the H chain variable region amino acid sequence described in SEQ ID NO: 9 and the L chain variable region amino acid sequence described in SEQ ID NO: 10;
(b3) the H chain variable region amino acid sequence described in SEQ ID NO: 21 and the L chain variable region amino acid sequence described in SEQ ID NO: 22.

12. A composition comprising the antibody according to any one of claims 2 to 11 and a pharmaceutically acceptable carrier.

13. The composition according to claim 12, wherein said composition is a pharmaceutical composition used for preventing and/or treating viral disease, malignant neoplasm, or immune disease.

14. The composition according to claim 13, wherein said viral disease is a disease that arises and/or progresses as a result of hepatitis C virus infection.

15. The composition according to claim 14, wherein the disease that arises and/or progresses as a result of hepatitis C virus infection is acute or chronic hepatitis C, cirrhosis, or liver cancer.

16. The composition according to claim 13, wherein said viral disease is a disease that arises and/or progresses as a result of hepatitis B virus infection.

17. The composition according to claim 16, wherein the disease that arises and/or progresses as a result of hepatitis B virus infection is acute or chronic hepatitis B, cirrhosis, or liver cancer.

18. The composition according to claim 13, wherein the malignant neoplasm is chronic myelocytic leukemia, malignant melanoma, multiple myeloma, renal cancer, gliosarcoma, medulloblastoma, astrocytoma, hairy cell leukemia, AIDS-related Kaposi's sarcoma, skin T lymphoma, or non-Hodgkin's lymphoma.

19. The composition according to claim 13, wherein the immune disease is multiple sclerosis.

20. A method for preventing and/or treating viral disease, malignant neoplasm, or immune disease, comprising the step of administering the antibody according to any one of claims 2 to 11, or the composition according to any one of claims 12 to 19.

21. Use of the antibody according to any one of claims 2 to 11 for producing the composition according to any one of claims 12 to 19.

22. A kit used in the method of preventing and/or treating diseases according to claim 20, wherein said kit comprises at least the antibody according to any one of claims 2 to 11, or the composition according to claim 12.

23. An antibody recognizing both an enzyme and a substrate thereof, wherein said antibody is a bispecific antibody which functionally substitutes for a cofactor that enhances the enzymatic reaction.

24. The antibody according to claim 23, wherein said enzyme is a proteolytic enzyme.

25. The antibody according to claim 24, wherein said proteolytic enzyme, substrate, and cofactor are blood coagulation/fibrinolysis associated factors.

26. The antibody according to claim 25, wherein the enzyme of a blood coagulation/fibrinolysis associated factor is blood coagulation factor IX and/or activated blood coagulation factor IX; the substrate is blood coagulation factor X; and the cofactor is blood coagulation factor VIII and/or activated blood coagulation factor VIII.

27. The antibody according to any one of claims 23 to 26, wherein said antibody comprises a complementarity determining region comprising the amino acid sequence of anti-blood coagulation factor IX/IXa antibody CDR3 of the following (a1) or (a2) or a complementarity determining region functionally equivalent thereto, and a complementarity determining region comprising the amino acid sequence of anti-blood coagulation factor X antibody CDR3 described in any one of the following (b1) to (b9) or a complementarity determining region functionally equivalent thereto:
(a1) H chain CDR3 amino acid sequence described in SEQ ID NO: 42;
(a2) H chain CDR3 amino acid sequence described in SEQ ID NO: 46;
(b1) H chain CDR3 amino acid sequence described in SEQ ID NO: 50;
(b2) H chain CDR3 amino acid sequence described in SEQ ID NO: 54;
(b3) H chain CDR3 amino acid sequence described in SEQ ID NO: 58;
(b4) H chain CDR3 amino acid sequence described in SEQ ID NO: 62;
(b5) H chain CDR3 amino acid sequence described in SEQ ID NO: 66;
(b6) H chain CDR3 amino acid sequence described in SEQ ID NO: 70;
(b7) H chain CDR3 amino acid sequence described in SEQ ID NO: 74;
(b8) H chain CDR3 amino acid sequence described in SEQ ID NO: 78;
(b9) H chain CDR3 amino acid sequence described in SEQ ID NO: 82.

28. The antibody according to any one of claims 23 to 26, wherein said antibody comprises a complementarity determining region comprising the amino acid sequences of anti-blood coagulation factor IX/IXa antibody CDR of the following (a1) or (a2) or a complementarity determining region functionally equivalent thereto, and a complementarity determining region comprising the amino acid sequence of anti-blood coagulation factor X antibody CDR described in any one of the following (b1) to (b9) or a complementarity determining region functionally equivalent thereto:
(a1) H chain CDR 1, 2, and 3 amino acid sequences described in SEQ ID NOs: 40, 41, and 42, respectively;
(a2) H chain CDR 1, 2, and 3 amino acid sequences described in SEQ ID NOs: 44, 45, and 46, respectively;
(b1) H chain CDR 1, 2, and 3 amino acid sequences described in SEQ ID NOs: 48, 49, and 50, respectively;
(b2) H chain CDR 1, 2, and 3 amino acid sequences described in SEQ ID NOs: 52, 53, and 54, respectively;
(b3) H chain CDR 1, 2, and 3 amino acid sequences described in SEQ ID NOs: 56, 57, and 58, respectively;
(b4) H chain CDR 1, 2, and 3 amino acid sequences described in SEQ ID NOs: 60, 61, and 62, respectively;
(b5) H chain CDR 1, 2, and 3 amino acid sequences described in SEQ ID NOs: 64, 65, and 66, respectively;
(b6) H chain CDR 1, 2, and 3 amino acid sequences described in SEQ ID NOs: 68, 69, and 70, respectively;
(b7) H chain CDR 1, 2, and 3 amino acid sequences described in SEQ ID NOs: 72, 73, and 74, respectively;
(b8) H chain CDR 1, 2, and 3 amino acid sequences described in SEQ ID NOs: 76, 77, and 78, respectively;
(b9) H chain CDR 1, 2, and 3 amino acid sequences described in SEQ ID NOs: 80, 81, and 82; respectively.

29. A composition comprising the antibody according to any one of claims 23 to 28 and a pharmaceutically acceptable carrier.

30. The composition according to claim 29, wherein said composition is a pharmaceutical composition used for preventing and/or treating bleeding, disorder accompanied by bleeding, or disorder caused by bleeding.

31. The composition according to claim 30, wherein the bleeding, disorder accompanied by bleeding, or disorder caused by bleeding is a disorder that arises and/or progresses as a result of an activity decrease or deficiency of blood coagulation factor VIII and/or activated blood coagulation factor VIII.

32. The composition according to claim 31, wherein the disorder that arises and/or progresses as a result of an activity decrease or deficiency of blood coagulation factor VIII and/or activated blood coagulation factor VIII is hemophilia A.

33. The composition according to claim 31, wherein the disorder that arises and/or progresses as a result of an activity decrease or deficiency of blood coagulation factor VIII and/or activated blood coagulation factor VIII is a disorder in which an inhibitor against blood coagulation factor VIII and/or activated blood coagulation factor VIII is generated.

34. The composition according to claim 31, wherein the disorder that arises and/or progresses as a result of an activity decrease or deficiency of blood coagulation factor VIII and/or activated blood coagulation factor VIII is acquired hemophilia.

35. The composition according to claim 31, wherein the disorder that arises and/or progresses as a result of an activity decrease of blood coagulation factor VIII and/or activated blood coagulation factor VIII is von Willerbrand's disease.

36. A method for preventing and/or treating bleeding, disorder accompanied by bleeding, or disorder caused by bleeding, wherein said method comprises the step of administering the antibody according to any one of claims 23 to 28, or the composition according to any one of claims 29 to 35.

37. Use of the antibody according to any one of claims 23 to 28 for preparing the composition according to any one of claims 29 to 35.

38. A kit used in the method of preventing and/or treating disorders according to claim 36, wherein said kit comprises at least the antibody according to any one of claims 23 to 28 or the composition according to claim 29.
